# DEMANDE DE BREVET EUROPEEN

(11) **EP 1 398 355 A1**
(43) Date de publication de la demande: **17.03.2004**
(21) Numéro de dépôt: 03292224.7
(22) Date de dépôt: 10.09.2003
(51) Int. Cl.: C09B 44/16, A61K 7/13, C09B 44/10

(54) **Composition pour la teinture des fibres kératiniques humaines comprenant un colorant monoazoique monocationique**

(30) Priorité: 10.09.2002 FR 0211186
(71) Demandeur: L'OREAL, 75008 Paris (FR)
(72) Inventeur: David, Hervé, 94340 Joinville Le Pont (FR); Berteuil, Nathalie, 91800 Brunoy (FR); Vidal, Laurent, 75013 Paris (FR)
(74) Mandataire: Wattremez, Catherine

(57) **Abrégé**

L'invention a pour objet une nouvelle composition pour la teinture des fibres kératiniques humaines et plus particulièrement des cheveux, comprenant un colorant monoazoïque monocationique de formule (I) **W1-N=N-W2-NW3-W4-W5**, les procédés de teinture la mettant en oeuvre, l'utilisation des colorants de formule (I) à titre de colorants directs, ainsi que les composés nouveaux de formule (I).

## Description

L'invention a pour objet une nouvelle composition pour la teinture des fibres kératiniques humaines et plus particulièrement des cheveux, contenant un colorant monoazoïque monocationique particulier, ainsi que les procédés de teinture des fibres mettant en oeuvre une telle composition. L'invention a aussi pour objet l'utilisation de ces colorants à titre de colorants directs, et les colorants monoazoïques monocationiques nouveaux.
Il est connu de teindre les fibres kératiniques humaines et en particulier les cheveux avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, donnent naissance par un processus de condensation oxydative à des composés colorés.
On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.
La variété des molécules mises en jeu au niveau des bases d'oxydation et des coupleurs permet l'obtention d'une riche palette de couleurs.
Ce procédé de coloration d'oxydation consiste à appliquer sur le fibres kératiniques, des bases d'oxydation ou un mélange de bases d'oxydation et de coupleurs avec un agent oxydant, par exemple de l'eau oxygénée, à laisser pauser, puis à rincer les fibres. Les colorations qui en résultent sont permanentes, puissantes, et résistantes aux agents extérieurs, notamment à la lumière, aux intempéries, aux lavages, à la transpiration et aux frottements. Généralement appliquées à pH basique, elles permettent d'obtenir une teinture et simultanément un éclaircissement de la fibre qui se traduit en pratique par la possibilité d'obtenir une coloration finale plus claire que la couleur d'origine. En outre, l'éclaircissement de la fibre a pour effet avantageux d'engendrer une couleur unie dans le cas des cheveux gris, et dans le cas de cheveux naturellement pigmentés, de faire ressortir la couleur, c'est à dire de la rendre plus visible.

Il est aussi connu de teindre les fibres kératiniques humaines par une coloration directe. Le procédé classiquement utilisé en coloration directe consiste à appliquer sur les fibres des colorants directs qui sont des molécules colorées et colorantes ayant une affinité pour les fibres, à laisser pauser, puis à rincer les fibres.
Il est connu par exemple d'utiliser des colorants directs nitrés benzéniques, anthraquinoniques, nitropyridiniques, des colorants azoïques, xanthéniques, acridiniques aziniques ou des colorants triarylméthaniques.
Les colorations qui en résultent sont des colorations particulièrement chromatiques qui sont cependant temporaires ou semi-permanentes car la nature des interactions qui lient les colorants directs à la fibre, et leur désorption de la surface et/ou du coeur de la fibre sont responsables de leur faible puissance tinctoriale et de leur mauvaise tenue aux lavages ou à la transpiration. Ces colorants directs sont en outre généralement sensibles à la lumière du fait de la faible résistance du chromophore vis-à-vis des attaques photochimiques et conduisent dans le temps à un affadissement de la coloration des cheveux. En outre, leur sensibilité à la lumière est dépendante de leur répartition uniforme ou en agrégats dans la fibre kératinique.
Il est connu d'utiliser des colorants directs en combinaison avec des agents oxydants. Cependant, les colorants directs sont généralement sensibles à l'action des agents oxydants tels que l'eau oxygénée, et les agents réducteurs tels que le bisulfite de sodium, ce qui les rend généralement difficilement utilisables dans les compositions de teinture directe éclaircissante à base d'eau oxygénée et à base d'un agent alcalinisant ou dans des compositions de teinture d'oxydation en association avec des précurseurs de colorants d'oxydation ou de coupleurs.
Par exemple, il a été proposé dans les demandes de brevets FR-1 584 965 et JP-062 711 435 de teindre les cheveux avec des compositions de teinture à base de colorants directs nitrés et/ou de colorants dispersés azoïques et d'eau oxygénée ammoniacale en appliquant sur les cheveux un mélange desdits colorants et dudit oxydant, réalisé juste avant l'emploi. Mais les colorations obtenues se sont révélées insuffisamment tenaces et disparaissent aux shampooings en laissant apparaître l'éclaircissement de la fibre capillaire. Une telle coloration devient inesthétique en évoluant au cours du temps.
On a également proposé dans les demandes de brevets JP-53 95693 et JP-55 022638 de teindre les cheveux avec des compositions à base de colorants directs cationiques de type oxazine et d'eau oxygénée ammoniacale, en appliquant sur les cheveux, dans une première étape, de l'eau oxygénée ammoniacale, puis dans une seconde étape, une composition à base du colorant direct oxazinique. Cette coloration n'est pas satisfaisante, en raison du fait qu'elle nécessite un procédé rendu trop lent par les temps de pause des deux étapes successives. Si par ailleurs on applique sur les cheveux un mélange extemporané du colorant direct oxazinique avec de l'eau oxygénée ammoniacale, on ne colore pas, ou du moins, on obtient une coloration de la fibre capillaire qui est presque inexistante.
Plus récemment, la demande de brevet FR 2 741 798 a décrit des compositions tinctoriales contenant des colorants directs azoïques ou azométhiniques comportant au moins un atome d'azote quaternisé, lesdites compositions étant à mélanger extemporanément à pH basique à une composition oxydante. Ces compositions permettent d'obtenir des colorations avec des reflets homogènes, tenaces et brillants. Cependant, elles ne permettent pas de teindre les fibres kératiniques humaines et notamment les cheveux avec autant de puissance qu'avec des compostions de coloration d'oxydation.
II existe donc un réel besoin de rechercher des colorants directs chromatiques qui permettent de teindre les fibres kératiniques humaines aussi puissamment que les colorants d'oxydation, qui soient aussi stables qu'eux à la lumière, soient également résistants aux intempéries, aux lavages et à la transpiration, et en outre, suffisamment stables en présence d'agents oxydants et réducteurs pour pouvoir obtenir simultanément un éclaircissement de la fibre, soit par utilisation de compositions directes éclaircissantes les contenant, soit par l'utilisation de compositions de coloration d'oxydation les contenant. Il existe aussi un réel besoin de rechercher des colorants directs qui permettent de teindre les fibres kératiniques humaines pour obtenir une gamme très large de couleurs, en particulier très chromatiques, sans oublier les nuances dites « fondamentale » comme les noirs et les marrons.

Ces buts sont atteints avec la présente invention qui a pour objet une composition pour la teinture des fibres kératiniques humaines, et plus particulièrement des cheveux, comprenant dans un milieu cosmétiquement acceptable, au moins un colorant monoazoïque monocationique de formule (I) suivante :

**W**_{**1**}**―N=N―W**_{**2**}**―NW**_{**3**}**―W**_{**4**}**―W**_{**5**} **(I)**

dans laquelle
- W₁ représente un hétérocycle aromatique cationique à 5 ou 6 chaînons de formules (II) et (III) suivantes : 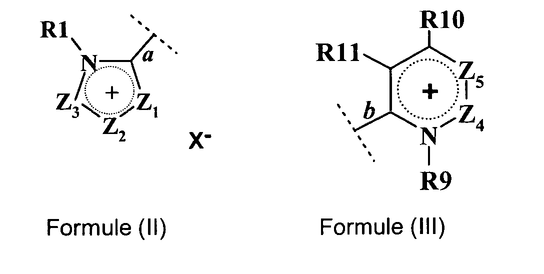
dans lesquelles :
- Z₁ représente un atome d'oxygène, un atome de soufre, un radical NR₂, ou un radical CR₃,
- Z₂ représente un atome d'azote ou un radical CR₄,
- Z₃ représente un radical NR₁₂ ou un radical CR₁₃,
- Z₄ représente un atome d'azote ou un radical CR₁₄,
- Z₅ représente un atome d'azote ou un radical CR₁₅,
Etant entendu que les formules (II) et (III) ne contiennent pas plus de deux hétéroatomes contigus ;
- la liaison a relie le cycle cationique à 5 chaînons de formule (II) à la fonction azoïque de la formule (I).
- la liaison b du cycle cationique à 6 chaînons de la formule (III) est reliée à la fonction azoïque de la formule (I).
- X- est un anion organique ou minéral.
- W₂ et W₄ (formule I) représentent, identiques ou différents, un groupe divalent aromatique carboné ou pyridinique de formules (IV) ou (V) suivantes :

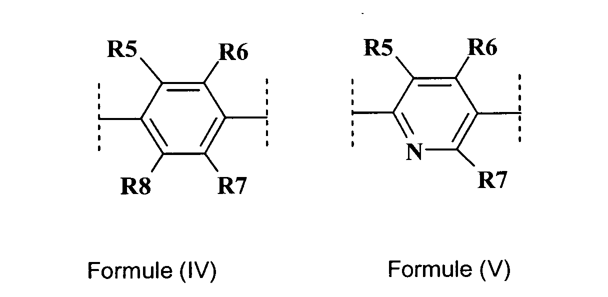

- W₃ représente un atome d'hydrogène ou un radical alkyle en C₁-C₆ éventuellement substitué par un ou plusieurs radicaux hydroxy, un ou plusieurs radicaux alcoxy, un ou plusieurs radicaux amino, un ou plusieurs radicaux monoalkylamino en C₁-C₄, un ou plusieurs radicaux dialkylamino en C₁-C₄.
- W₅ représente un radical hétéroaromatique azoté à 5 chaînons relié à W₄ par l'atome d'azote du cycle dudit radical hétéroaromatique, ce radical hétéroaromatique étant choisi parmi les radicaux pyrazolyle, pyrrolyle, imidazolyle, triazolyle, thiadiazolyle, chacun de ces radicaux hétéroaromatiques pouvant être substitué par un ou plusieurs atomes d'hydrogène, de chlore ou de fluor, ou par un ou plusieurs radicaux alkyle en C₁-C₆, éventuellement substitués par un ou plusieurs radicaux hydroxy, alcoxy en C₁-C₄, (poly)-hydroxyalcoxy en C₂-C₄, amino, (di)alkylamino en C₁-C₄, (poly)hydroxyalkylamino en C₂-C₄, carboxyle, sulfonyle, alcoxycarbonyle en C₁-C₄, thioether en C₁-C₄ ; ou par un ou plusieurs radicaux phényle éventuellement substitués par un ou plusieurs radicaux choisis parmi les radicaux hydroxy, alcoxy en C₁-C₂, amino, (di)-alkylamino en C₁-C₂, carboxy, sulfonyle, alkyle en C₁-C₄, halogène, thioéther en C₁-C₂,
- R₁, R₂, R₉, R₁₂ représentent, identiques ou différents, un radical phényle éventuellement substitué ou un radical alkyle en C₁-C₈, éventuellement substitué par un ou plusieurs radicaux choisis dans le groupe formé par les radicaux hydroxy, alcoxy en C₁-C₂, (poly)-hydroxyalcoxy en C₂-C₄, amino, (di)alkylamino en C₁-C₂,
- R₅, R₆, R₇ et R₈ représentent, identiques ou différents, un atome d'hydrogène ; un atome de chlore ; un atome de brome ; une chaîne hydrocarbonée en C₁-C₈ linéaire ou ramifiée, pouvant former un ou plusieurs cycles carbonés comportant de 3 à 6 chaînons, et pouvant être saturée ou insaturée, dont un ou plusieurs atomes de carbone de la chaîne carbonée peuvent être remplacés par un atome d'oxygène, d'azote ou de soufre ou par un groupement SO₂ et dont les atomes de carbone peuvent être, indépendamment les uns des autres, substitués par un ou plusieurs atomes d'halogène ; R₅, R₆, R₇ et R₈ ne comportant pas de liaison peroxyde, ni de radicaux diazo ou nitroso.
- R₃, R₄, Rio, R₁₁, R₁₃, R₁₄ et R₁₅ représentent, identiques ou différents, un atome d'hydrogène, une chaîne hydrocarbonée en C₁-C₁₆, de préférence en C₁-C₈, linéaire ou ramifiée, pouvant former un ou plusieurs cycles carbonés comportant de 3 à 6 chaînons, et pouvant être saturée ou insaturée, dont un ou plusieurs atomes de carbone de la chaîne carbonée peuvent être remplacés par un atome d'oxygène, d'azote ou de soufre ou par un groupement SO₂, et dont les atomes de carbone peuvent être, indépendamment les uns des autres, substitués par un ou plusieurs atomes d'halogène ; R₃, R₄, R₁₀, R₁₁, R₁₃, R₁₄ et R₁₅ ne comportant pas de liaison peroxyde, ni de radicaux diazo ou nitroso.
- R₄ avec R₁₃ et R₁₄ avec R₁₅ peuvent former un cycle aromatique carboné, tel qu'un phényle.

Selon l'invention, lorsqu'il est indiqué qu'un ou plusieurs des atomes de carbone de la chaîne hydrocarbonée définie pour les radicaux R₃, R₄, R₅, R₆, R₇, R₈, R₁₀, R₁₁, R₁₃, R₁₄ et R₁₅ peuvent être remplacés par un atome d'oxygène, d'azote ou de soufre ou par un groupement SO₂, et/ou que ces chaînes hydrocarbonées sont insaturées, cela signifie que l'on peut, à titre d'exemple, faire les transformations suivantes :

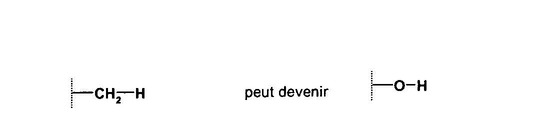

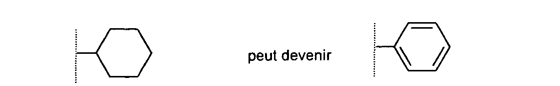

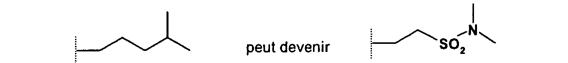

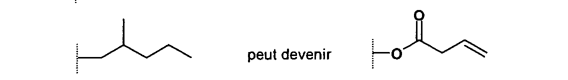

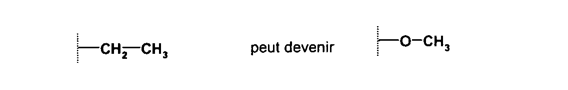

En particulier, on entend par "chaîne hydrocarbonée ramifiée", une chaîne pouvant former un ou plusieurs cycles carbonés comportant de 3 à 6 chaînons. On entend par chaîne hydrocarbonée insaturée, une chaîne pouvant comporter une ou plusieurs liaisons doubles et/ou une ou plusieurs liaisons triples, cette chaîne hydrocarbonée pouvant conduire à des groupements aromatiques.

X- représente un anion organique ou minéral cosmétiquement acceptable par exemple choisi parmi un halogénure tel que chlorure, bromure, fluorure, iodure ; un hydroxyde ; un sulfate ; un hydrogénosulfate ; un alkyl(C₁-C₆)sulfate tel que par exemple un méthylsulfate ou un éthylsulfate ; un acétate ; un tartrate ; un oxalate ; un alkyl(C₁-C₆)sulfonate tel que méthylsulfonate ; un arylsulfonate substitué ou non substitué par un radical alkyle en C₁-C₄ tel que par exemple un 4-toluylsulfonate.
De préférence selon l'invention, W₃ (formule I) représente un atome d'hydrogène ou un radical alkyle en C₁-C₄ éventuellement substitué par un ou plusieurs radicaux choisis parmi les radicaux hydroxy, alcoxy en C₁-C₂, amino, (di)alkylamino en C₁-C₂.
Selon un mode de réalisation particulièrement préféré, W₃ (formule I) représente un atome d'hydrogène ou un radical méthyle, éthyle, 2-hydroxyéthyle, et encore plus particulièrement un atome d'hydrogène.
De préférence selon l'invention, W₅ (formule I) est choisi parmi les cycles pyrazolyle, pyrrolyle, imidazolyle, et plus particulièrement encore parmi les cycles pyrrolyle, imidazolyle.
Selon un mode de réalisation particulier, W₅ (formule I) est un cycle hétéroaromatique azoté à 5 chaînons substitué par un ou plusieurs radicaux alkyle en C₁-C₆ éventuellement substitués par un hydroxy, alcoxy en C₁-C₄, amino, mono ou dialkylamino en C₁-C₄; un atome de chlore ou de fluor ; un radical alcoxycarbonyle en C₁-C₄ ; un radical phényle éventuellement substitué par un hydroxy, alcoxy en C₁-C₂, amino, mono ou dialkylamino en C₁-C₂, un atome de brome ou de chlore ; un radical dialkylamino en C₁-C₂, un radical alcoxy en C₁-C₂.
W₅ (formule I) est plus particulièrement choisi parmi les radicaux pyrrolyle, imidazolyle qui sont éventuellement substitués par un à deux radicaux méthyle, éthyle, propyle, phényle, 4-chlorophényle, éthoxycarbonyle.
Parmi les substituants, R₁, R₂, R₉, R₁₂ représentent préférentiellement, identiques ou différents, un radical alkyle en C₁-C₄ éventuellement substitué par un ou plusieurs radicaux choisis parmi les radicaux hydroxy, alcoxy en C₁-C₂, amino, (di)alkylamino en C₁-C₂.
Selon un mode de réalisation particulièrement préféré, R₁, R₂, R₉, R₁₂ représentent, identiques ou différents, un radical méthyle, éthyle, propyle, 2-hydroxyéthyle.
Parmi les substituants, R₅, R₆, R₇ et R₈ sont de préférence choisis, identiques ou différents, parmi un atome d'hydrogène, un radical méthyle, éthyle, isopropyle, méthoxyméthyle, hydroxyméthyle, 1-carboxyméthyle, 1-aminométhyle, 2-carboxyéthyle, 2-hydroxyéthyle, 3-hydroxypropyle, 1,2-dihydroxyéthyle, 1-hydroxy-2-aminoéthyle, 2-hydroxy-1-aminoéthyle, méthoxy, éthoxy, 2-hydroxyéthyloxy, 2-aminoéthyloxy.
Selon un mode de réalisation particulièrement préféré, R₅, R₆, R₇ et R₈ sont choisis, identiques ou différents, parmi un atome d'hydrogène, un radical méthyle, hydroxyméthyle, 2-hydroxyéthyle, 1,2-dihydroxyéthyle, méthoxy, 2-hydroxyéthyloxy, et encore plus préférentiellement parmi un atome d'hydrogène, un radical méthyle, un radical méthoxy.
Parmi les substituants, R₃, R₄, R₁₀, R₁₁, R₁₃, R₁₄ et R₁₅, identiques ou différents, sont de préférence choisis parmi un atome d'hydrogène ; un radical alkyle en C₁-C₄ linéaire ou ramifié, éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁-C₂ ; un radical phényle éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁-C₂, ou un atome d'halogène tel que chlore, fluor ou brome ; un radical sulfonylamino ; un radical alcoxy en C₁-C₂ ; un radical (poly)-hydroxyalcoxy en C₂-C₄ ; un radical amino ; un radical (di)alkylamino en C₁-C₂ ; un radical (poly)-hydroxyalkylamino en C₂-C₄.
Plus préférentiellement, R₃, R₄, R₁₀, R₁₁, R₁₃, R₁₄ et R₁₅ représentent, identiques ou différents, un atome d'hydrogène, un radical alkyle en C₁-C₄ éventuellement substitué par un ou plusieurs radicaux choisis parmi les radicaux hydroxy, amino, (di)alkylamino en C₁-C₂ ; un radical alcoxy en C₁-C₂ ; un radical amino ; un radical (di)alkylamino en C₁-C₂ ; un radical (poly)-hydroxyalkylamino en C₂-C₄.
Selon un mode de réalisation particulièrement préféré, R₃, R₄, R₁₀, R₁₁, R₁₃, R₁₄ et R₁₅ représentent, identiques ou différents, un atome d'hydrogène, un radical méthyle, phényle, 2-hydroxyméthyle, méthoxy, éthoxy, 2-hydroxyéthyloxy, amino, méthylamino, diméthylamino, 2-hydroxyéthylamino.

De préférence, selon l'invention, dans la formule (II), Z₁ désigne NR₂.
De préférence, selon l'invention, dans la formule (II), Z₂ désigne CR₄.
De préférence, selon l'invention, dans la formule (II), Z₃ désigne CR₁₃.
De préférence, selon l'invention, dans la formule (III), Z₄ désigne CR₁₄.
De préférence, selon l'invention, dans la formule (III), Z₅ désigne CR₁₅.

Parmi les colorants monoazoïques monocationiques de formule (I), on peut citer notamment de préférence, les:
2-(4-amino-N-(4-(N-pyrrolophényl))-phénylazo)-1,3-diméthyl-3H-imidazol-1-ium,
2-(4-amino-N-(4-(N-(2,5-diméthyl)-pyrrolophényl))-phénylazo)-1,3-diméthyl-3H-imidazol-1-ium,
2-(4-amino-N-(4-(N-(2,5-diéthyl)-pyrrolophényl))-phénylazo)-1,3-diméthyl-3H-imidazol-1-ium,
2-(4-amino-N-(4-(N-(2,5-propyl)-pyrrolophényl))-phénylazo)-1,3-diméthyl-3H-imidazol-1-ium,
2-(4-amino-N-(4-(N-(2-methyl-5-ethyl)-pyrrolophényl))-phénylazo)-1,3-diméthyl-3H-imidazol-1-ium,
2-(4-amino-N-(4-(N-(2-méthyl-5-propyl)-pyrrolophényl))-phénylazo)-1,3-diméthyl-3H-imidazol-1-ium,
2-(4-amino-N-(4-(N-(2-méthyl-5-phényl)-pyrrolophényl))-phénylazo)-1,3-diméthyl-3H-imidazol-1-ium,
2-(4-amino-N-(4-(N-[2-méthyl-3-carboxyéthyl-5-(4-chlorophényl)-pyrrolophényl)]-phénylazo)-1,3-diméthyl-3H-imidazol-1-ium.
2-(4-amino-N-(4-(N-[2-5-(4-fluorophényl)-pyrrolophényl)]-phénylazo)-1,3-diméthyl-3H-imidazol-1-ium.
2-(4-amino-N-(4-(N-[2-méthyl-5-(4-chlorophényl)-pyrrolophényl)]-phénylazo)-1,3-diméthyl-3H-imidazol-1-ium.
2-(4-amino-N-(4-(N-[2-tertbutyl-5-carboxyethyl)-pyrrolophényl)]-phénylazo)-1,3-diméthyl-3H-imidazol-1-ium.
2-(4-amino-N-(4-(N-[2-méthyl-4-carboxyethyl-5-phényl)-pyrrolophényl)]-phénylazo)-1, 3-diméthyl-3H-imidazol-1-ium.
2-(4-amino-N-(4-(N-[2-méthyl-4-methyl-5-(4-methoxyphényl))-pyrrolophényl)]-phénylazo)-1,3-diméthyl-3H-imidazol-1-ium.
2-(4-amino-N-(4-(N-[2,5-diméthyl-3,4-di-isopropyl)-pyrrolophényl)]-phénylazo)-1,3-diméthyl-3H-imidazol-1-ium.
2-(4-amino-N-(4-(N-pyrrolophényl))-phénylazo)-1,3-diéthyl-3H-imidazol-1-ium,
2-(4-amino-N-(4-(N-(2,5-diméthyl)-pyrrolophényl))-phénylazo)-1,3-diéthyl -3H-imidazol-1-ium,
2-(4-amino-N-(4-(N-(2,5-diéthyl)-pyrrolophényl))-phénylazo)-1,3-diéthyl-3H-imidazol-1-ium,
2-(4-amino-N-(4-(N-(2,5-propyl)-pyrrolophényl))-phénylazo)-1,3-diéthyl-3H-imidazol-1-ium,
2-(4-amino-N-(4-(N-(2-methyl-5-ethyl)-pyrrolophényl))-phénylazo)-1,3-diéthyl-3H-imidazol-1-ium,
2-(4-amino-N-(4-(N-(2-méthyl-5-propyl)-pyrrolophényl))-phénylazo)-1,3-diéthyl-3H-imidazol-1-ium,
2-(4-amino-N-(4-(N-(2-méthyl-5-phényl)-pyrrolophényl))-phénylazo)-1,3-diéthyl-3H-imidazol-1-ium,
2-(4-amino-N-(4-(N-pyrrolophényl))-phénylazo)-1,3-dipropyl-3H-imidazol-1-ium,
2-(4-amino-N-(4-(N-(2,5-diméthyl)-pyrrolophényl))-phénylazo)-1,3-dipropyl-3H-imidazol-1-ium,
2-(4-amino-N-(4-(N-(2,5-diéthyl)-pyrrolophényl))-phénylazo)-1,3-dipropyl-3H-imidazol-1-ium,
2-(4-amino-N-(4-(N-(2,5-propyl)-pyrrolophényl))-phénylazo)-1,3-dipropyl-3H-imidazol-1-ium,
2-(4-amino-N-(4-(N-(2-methyl-5-ethyl)-pyrrolophényl))-phénylazo)-1,3-dipropyl-3H-imidazol-1-ium,
2-(4-amino-N-(4-(N-(2-méthyl-5-propyl)-pyrrolophényl))-phénylazo)-1,3-dipropyl-3H-imidazol-1-ium,
2-(4-amino-N-(4-(N-(2-méthyl-5-phényl)-pyrrolophényl))-phénylazo)-1,3-dipropyl-3H-imidazol-1-ium,
2-(4-amino-N-(4-(N-pyrrolophényl))-phénylazo)-1,3-di(2-hydroxyethyl)-3H-imidazol-1-ium,
2-(4-amino-N-(4-(N-(2,5-diméthyl)-pyrrolophényl))-phénylazo)-1,3-di(2-hydroxyethyl)-3H-imidazol-1-ium,
2-(4-amino-N-(4-(N-(2,5-diéthyl)-pyrrolophényl))-phénylazo)-1,3-di(2-hydroxyethyl)-3H-imidazol-1-ium,
2-(4-amino-N-(4-(N-(2,5-propyl)-pyrrolophényl))-phénylazo)-1,3-di(2-hydroxyethyl)-3H-imidazol-1-ium,
2-(4-amino-N-(4-(N-(2-methyl-5-ethyl)-pyrrolophényl))-phénylazo)-1,3-di(2-hydroxyethyl)-3H-imidazol-1-ium,
2-(4-amino-N-(4-(N-(2-méthyl-5-propyl)-pyrrolophényl))-phénylazo)-1,3-di(2-hydroxyethyl)-3H-imidazol-1-ium,
2-(4-amino-N-(4-(N-(2-méthyl-5-phényl)-pyrrolophényl))-phénylazo)-1,3-di(2-hydroxyethyl)-3H-imidazol-1-ium,
2-(4-amino-N-(4-(N-pyrrolophényl))-phénylazo)-4-phényl-1,3-diméthyl-3H-imidazol-1-ium,
2-(4-amino-N-(4-(N-(2,5-diméthyl)-pyrrolophényl))-phénylazo)-4-phényl-1,3-diméthyl-3H-imidazol-1-ium,
2-(4-amino-N-(4-(N-(2,5-diéthyl)-pyrrolophényl))-phénylazo)-4-phényl-1,3-diméthyl-3H-imidazol-1-ium,
2-(4-amino-N-(4-(N-(2,5-propyl)-pyrrolophényl))-phénylazo)-4-phényl-1,3-diméthyl-3H-imidazol-1-ium,
2-(4-amino-N-(4-(N-(2-methyl-5-ethyl)-pyrrolophényl))-phénylazo)-4-phényl-1,3-diméthyl-3H-imidazol-1-ium,
2-(4-amino-N-(4-(N-(2-méthyl-5-propyl)-pyrrolophényl))-phénylazo)-4-phényl-1,3-diméthyl-3H-imidazol-1-ium,
2-(4-amino-N-(4-(N-(2-méthyl-5-phényl)-pyrrolophényl))-phénylazo)-4-phényl-1,3-diméthyl-3H-imidazol-1-ium,
2-(4-amino-N-(4-(N-[2-méthyl-3-carboxyéthyl-5-(4-chlorophényl)-pyrrolophényl)]-phénylazo)-4-phény)-1,3-diméthyl-3H-imidazol-1-ium.
2-(4-amino-N-(4-(N-[2-5-(4-fluorophényl)-pyrrolophényl)]-phénylazo)-4-phényl-1,3-diméthyl-3H-imidazol-1-ium.
2-(4-amino-N-(4-(N-[2-méthyl-5-(4-chlorophényl)-pyrrolophényl)]-phénylazo)-4-phényl-1,3-diméthyl-3H-imidazol-1-ium.
2-(4-amino-N-(4-(N-[2-tertbutyl-5-carboxyethyl)-pyrrolophényl)]-phénylazo)-4-phényl-1,3-diméthyl-3H-imidazol-1-ium.
2-(4-amino-N-(4-(N-[2-méthyl-4-carboxyethyl-5-phényl)-pyrrolophényl)]-phénylazo)-4-phényl-1,3-diméthyl-3H-imidazol-1-ium.
2-(4-amino-N-(4-(N-[2-méthyl-4-methyl-5-(4-methoxyphényl))-pyrrolophényl)]-phénylazo)-4-phényl-1,3-diméthyl-3H-imidazol-1-ium.
2-(4-amino-N-(4-(N-[2,5-diméthyl-3,4-di-isopropyl)-pyrrolophényl)]-phénylazo)-4-phényl-1,3-diméthyl-3H-imidazol-1-ium.
2-(4-amino-N-(4-(N-pyrrolophényl))-phénylazo)-4-phényl-1,3-diéthyl-3H-imidazol-1-ium,
2-(4-amino-N-(4-(N-(2,5-diméthyl)-pyrrolophényl))-phénylazo)-4-phényl-1,3-diéthyl -3H-imidazol-1-ium,
2-(4-amino-N-(4-(N-(2,5-diéthyl)-pyrrolophényl))-phénylazo)-4-phényl-1,3-diéthyl-3H-imidazol-1-ium,
2-(4-amino-N-(4-(N-(2,5-propyl)-pyrrolophényl))-phénylazo)-4-phényl-1,3-diéthyl-3H-imidazol-1-ium,
2-(4-amino-N-(4-(N-(2-methyl-5-ethyl)-pyrrolophényl))-phénylazo)-4-phényl-1,3-diéthyl-3H-imidazol-1-ium,
2-(4-amino-N-(4-(N-(2-méthyl-5-propyl)-pyrrolophényl))-phénylazo)-4-phényl-1,3-diéthyl-3H-imidazol-1-ium,
2-(4-amino-N-(4-(N-(2-méthyl-5-phényl)-pyrrolophényl))-phénylazo)-4-phényl-1,3-diéthyl-3H-imidazol-1-ium,
2-(4-amino-N-(4-(N-pyrrolophényl))-phénylazo)-4-phényl-1,3-dipropyl-3H-imidazol-1-ium,
2-(4-amino-N-(4-(N-(2,5-diméthyl)-pyrrolophényl))-phénylazo)-4-phényl-1,3-dipropyl-3H-imidazol-1-ium,
2-(4-amino-N-(4-(N-(2,5-diéthyl)-pyrrolophényl))-phénylazo)-4-phényl-1,3-dipropyl-3H-imidazol-1-ium,
2-(4-amino-N-(4-(N-(2,5-propyl)-pyrrolophényl))-phénylazo)-4-phényl-1,3-dipropyl-3H-imidazol-1-ium,
2-(4-amino-N-(4-(N-(2-methyl-5-ethyl)-pyrrolophényl))-phénylazo)-4-phényl-1,3-dipropyl-3H-imidazol-1-ium,
2-(4-amino-N-(4-(N-(2-méthyl-5-propyl)-pyrrolophényl))-phénylazo)-4-phényl-1,3-dipropyl-3H-imidazol-1-ium,
2-(4-amino-N-(4-(N-(2-méthyl-5-phényl)-pyrrolophényl))-phénylazo)-4-phényl-1,3-dipropyl-3H-imidazol-1-ium,
2-(4-amino-N-(4-(N-pyrrolophényl))-phénylazo)-4-phényl-1,3-di(2-hydroxyethyl)-3H-imidazol-1-ium,
2-(4-amino-N-(4-(N-(2,5-diméthyl)-pyrrolophényl))-phénylazo)-4-phényl-1,3-di(2-hydroxyethyl)-3H-imidazol-1-ium,
2-(4-amino-N-(4-(N-(2,5-diéthyl)-pyrrolophényl))-phénylazo)-4-phényl-1,3-di(2-hydroxyethyl)-3H-imidazol-1-ium,
2-(4-amino-N-(4-(N-(2,5-propyl)-pyrrolophényl))-phénylazo)-4-phényl-1,3-di(2-hydroxyethyl)-3H-imidazol-1-ium,
2-(4-amino-N-(4-(N-(2-methyl-5-ethyl)-pyrrolophényl))-phénylazo)-4-phényl-1,3-di(2-hydroxyethyl)-3H-imidazol-1-ium,
2-(4-amino-N-(4-(N-(2-méthyl-5-propyl)-pyrrolophényl))-phénylazo)-4-phényl-1,3-di(2-hydroxyethyl)-3H-imidazol-1-ium,
2-(4-amino-N-(4-(N-(2-méthyl-5-phényl)-pyrrolophényl))-phénylazo)-4-phényl-1,3-di(2-hydroxyethyl)-3H-imidazol-1-ium,
2-(4-amino-N-(4-(N-pyrrolophényl))-phénylazo)-4-méthyl-1,3-diméthyl-3H-imidazol-1-ium,
2-(4-amino-N-(4-(N-(2,5-diméthyl)-pyrrolophényl))-phénylazo)-4-méthyl-1,3-diméthyl-3H-imidazol-1-ium,
2-(4-amino-N-(4-(N-(2,5-diéthyl)-pyrrolophényl))-phénylazo)-4-méthyl-1,3-diméthyl-3H-imidazol-1-ium,
2-(4-amino-N-(4-(N-(2,5-propyl)-pyrrolophényl))-phénylazo)-4-méthyl-1,3-diméthyl-3H-imidazol-1-ium,
2-(4-amino-N-(4-(N-(2-methyl-5-ethyl)-pyrrolophényl))-phénylazo)-4-méthyl-1,3-diméthyl-3H-imidazol-1-ium,
2-(4-amino-N-(4-(N-(2-méthyl-5-propyl)-pyrrolophényl))-phénylazo)-4-méthyl-1,3-diméthyl-3H-imidazol-1-ium,
2-(4-amino-N-(4-(N-(2-méthyl-5-phényl)-pyrrolophényl))-phénylazo)-4-méthyl-1, 3-diméthyl-3H-imidazol-1-ium,
2-(4-amino-N-(4-(N-[2-méthyl-3-carboxyéthyl-5-(4-chlorophényl)-pyrrolophényl)]-phénylazo)-4-méthyl-1,3-diméthyl-3H-imidazol-1-ium.
2-(4-amino-N-(4-(N-[2-5-(4-fluorophényl)-pyrrolophényl)]-phénylazo)-4-méthyl-1,3-diméthyl-3H-imidazol-1-ium.
2-(4-amino-N-(4-(N-[2-méthyl-5-(4-chlorophényl)-pyrrolophényl)]-phénylazo)-4-méthyl-1,3-diméthyl-3H-imidazol-1-ium.
2-(4-amino-N-(4-(N-[2-tertbutyl-5-carboxyethyl)-pyrrolophényl)]-phénylazo)-4-méthyl-1,3-diméthyl-3H-imidazol-1-ium.
2-(4-amino-N-(4-(N-[2-méthyl-4-carboxyethyl-5-phényl)-pyrrolophényl)]-phénylazo)-4-méthyl-1,3-diméthyl-3H-imidazol-1-ium.
2-(4-amino-N-(4-(N-[2-méthyl-4-methyl-5-(4-methoxyphényl))-pyrrolophényl)]-phénylazo)-4-méthyl-1,3-diméthyl-3H-imidazol-1-ium.
2-(4-amino-N-(4-(N-[2,5-diméthyl-3,4-di-isopropyl)-pyrrolophényl)]-phénylazo)-4-méthyl-1,3-diméthyl-3H-imidazol-1-ium.
2-(4-amino-N-(4-(N-pyrrolophényl))-phénylazo)-4-méthyl-1,3-diéthyl-3H-imidazol-1-ium,
2-(4-amino-N-(4-(N-(2,5-diméthyl)-pyrrolophényl))-phénylazo)-4-méthyl-1,3-diéthyl-3H-imidazol-1-ium,
2-(4-amino-N-(4-(N-(2,5-diéthyl)-pyrrolophényl))-phénylazo)-4-méthyl-1,3-diéthyl-3H-imidazol-1-ium,
2-(4-amino-N-(4-(N-(2,5-propyl)-pyrrolophényl))-phénylazo)-4-méthyl-1,3-diéthyl-3H-imidazol-1-ium,
2-(4-amino-N-(4-(N-(2-methyl-5-ethyl)-pyrrolophényl))-phénylazo)-4-méthyl-1,3-diéthyl-3H-imidazol-1-ium,
2-(4-amino-N-(4-(N-(2-méthyl-5-propyl)-pyrrolophényl))-phénylazo)-4-méthyl-1,3-diéthyl-3H-imidazol-1-ium,
2-(4-amino-N-(4-(N-(2-méthyl-5-phényl)-pyrrolophényl))-phénylazo)-4-méthyl-1,3-diéthyl-3H-imidazol-1-ium,
2-(4-amino-N-(4-(N-pyrrolophényl))-phénylazo)-4-méthyl-1,3-dipropyl-3H-imidazol-1-ium,
2-(4-amino-N-(4-(N-(2,5-diméthyl)-pyrrolophényl))-phénylazo)-4-méthyl-1,3-dipropyl-3H-imidazol-1-ium,
2-(4-amino-N-(4-(N-(2,5-diéthyl)-pyrrolophényl))-phénylazo)-4-méthyl-1,3-dipropyl-3H-imidazol-1-ium,
2-(4-amino-N-(4-(N-(2,5-propyl)-pyrrolophényl))-phénylazo)-4-méthyl-1,3-dipropyl-3H-imidazol-1-ium,
2-(4-amino-N-(4-(N-(2-methyl-5-ethyl)-pyrrolophényl))-phénylazo)-4-méthyl-1,3-dipropyl-3H-imidazol-1-ium,
2-(4-amino-N-(4-(N-(2-méthyl-5-propyl)-pyrrolophényl))-phénylazo)-4-méthyl-1,3-dipropyl-3H-imidazol-1-ium,
2-(4-amino-N-(4-(N-(2-méthyl-5-phényl)-pyrrolophényl))-phénylazo)-4-méthyl-1,3-dipropyl-3H-imidazol-1-ium,
2-(4-amino-N-(4-(N-pyrrolophényl))-phénylazo)-4-méthyl-1,3-di(2-hydroxyethyl)-3H-imidazol-1-ium,
2-(4-amino-N-(4-(N-(2,5-diméthyl)-pyrrolophényl))-phénylazo)-4-méthyl-1,3-di(2-hydroxyethyl)-3H-imidazol-1-ium,
2-(4-amino-N-(4-(N-(2,5-diéthyl)-pyrrolophényl))-phénylazo)-4-méthyl-1,3-di(2-hydroxyethyl)-3H-imidazol-1-ium,
2-(4-amino-N-(4-(N-(2,5-propyl)-pyrrolophényl))-phénylazo)-4-méthyl-1,3-di(2-hydroxyethyl)-3H-imidazol-1-ium,
2-(4-amino-N-(4-(N-(2-methyl-5-ethyl)-pyrrolophényl))-phénylazo)-4-méthyl-1,3-di(2-hydroxyethyl)-3H-imidazol-1-ium,
2-(4-amino-N-(4-(N-(2-méthyl-5-propyl)-pyrrolophényl))-phénylazo)-4-méthyl-1,3-di(2-hydroxyethyl)-3H-imidazol-1-ium,
2-(4-amino-N-(4-(N-(2-méthyl-5-phényl)-pyrrolophényl))-phénylazo)-4-méthyl-1,3-di(2-hydroxyethyl)-3H-imidazol-1-ium,
2-(4-amino-N-(4-(N-pyrrolophényl))-phénylazo)-4-hydroxyméthyl-1,3-diméthyl-3H-imidazol-1-ium,
2-(4-amino-N-(4-(N-(2,5-diméthyl)-pyrrolophényl))-phénylazo)-4-hydroxyméthyl-1,3-diméthyl-3H-imidazol-1-ium,
2-(4-amino-N-(4-(N-(2,5-diéthyl)-pyrrolophényl))-phénylazo)-4-hydroxyméthyl-1,3-diméthyl-3H-imidazol-1-ium,
2-(4-amino-N-(4-(N-(2,5-propyl)-pyrrolophényl))-phénylazo)-4-hydroxyméthyl-1,3-diméthyl-3H-imidazol-1-ium,
2-(4-amino-N-(4-(N-(2-methyl-5-ethyl)-pyrrolophényl))-phénylazo)-4-hydroxyméthyl-1,3-diméthyl-3H-imidazol-1-ium,
2-(4-amino-N-(4-(N-(2-méthyl-5-propyl)-pyrrolophényl))-phénylazo)-4-hydroxyméthyl-1,3-diméthyl-3H-imidazol-1-ium,
2-(4-amino-N-(4-(N-(2-méthyl-5-phényl)-pyrrolophényl))-phénylazo)-4-hydroxyméthyl-1,3-diméthyl-3H-imidazol-1-ium,
2-(4-amino-N-(4-(N-[2-méthyl-3-carboxyéthyl-5-(4-chlorophényl)-pyrrolophényl)]-phénylazo)-4-hydroxyméthyl-1,3-diméthyl-3H-imidazol-1-ium.
2-(4-amino-N-(4-(N-[2-5-(4-fluorophényl)-pyrrolophényl)]-phénylazo)-4-hydroxyméthyl-1,3-diméthyl-3H-imidazol-1-ium.
2-(4-amino-N-(4-(N-[2-méthyl-5-(4-chlorophényl)-pyrrolophényl)]-phénylazo)-4-hydroxyméthyl-1,3-diméthyl-3H-imidazol-1-ium.
2-(4-amino-N-(4-(N-[2-tertbutyl-5-carboxyethyl)-pyrrolophényl)]-phénylazo)-4-hydroxyméthyl-1,3-diméthyl-3H-imidazol-1-ium.
2-(4-amino-N-(4-(N-[2-méthyl-4-carboxyethyl-5-phényl)-pyrrolophényl)]-phénylazo)-4-hydroxyméthyl-1,3-diméthyl-3H-imidazol-1-ium.
2-(4-amino-N-(4-(N-[2-méthyl-4-methyl-5-(4-methoxyphényl))-pyrrolophényl)]-phénylazo)-4-hydroxyméthyl-1,3-diméthyl-3H-imidazol-1-ium.
2-(4-amino-N-(4-(N-[2,5-diméthyl-3,4-di-isopropyl)-pyrrolophényl)]-phénylazo)-4-hydroxyméthyl-1,3-diméthyl-3H-imidazol-1-ium.
2-(4-amino-N-(4-(N-pyrrolophényl))-phénylazo)-4-hydroxyméthyl-1,3-diéthyl-3H-imidazol-1-ium,
2-(4-amino-N-(4-(N-(2,5-diméthyl)-pyrrolophényl))-phénylazo)-4-hydroxyméthyl-1,3-diéthyl- 3H-imidazol-1-ium,
2-(4-amino-N-(4-(N-(2,5-diéthyl)-pyrrolophényl))-phénylazo)-4-hydroxyméthyl-1,3-diéthyl-3H-imidazol-1-ium,
2-(4-amino-N-(4-(N-(2,5-propyl)-pyrrolophényl))-phénylazo)-4-hydroxyméthyl-1,3-diéthyl-3H-imidazol-1-ium,
2-(4-amino-N-(4-(N-(2-methyl-5-ethyl)-pyrrolophényl))-phénylazo)-4-hydroxyméthyl-1,3-diéthyl-3H-imidazol-1-ium,
2-(4-amino-N-(4-(N-(2-méthyl-5-propyl)-pyrrolophényl))-phénylazo)-4-hydroxyméthyl-1,3-diéthyl-3H-imidazol-1-ium,
2-(4-amino-N-(4-(N-(2-méthyl-5-phényl)-pyrrolophényl))-phénylazo)-4-hydroxyméthyl-1,3-diéthyl-3H-imidazol-1-ium,
2-(4-amino-N-(4-(N-pyrrolophényl))-phénylazo)-4-hydroxyméthyl-1,3-dipropyl-3H-imidazol-1-ium,
2-(4-amino-N-(4-(N-(2,5-diméthyl)-pyrrolophényl))-phénylazo)-4-hydroxyméthyl-1,3-dipropyl-3H-imidazol-1-ium,
2-(4-amino-N-(4-(N-(2,5-diéthyl)-pyrrolophényl))-phénylazo)-4-hydroxyméthyl-1,3-dipropyl-3H-imidazol-1-ium,
2-(4-amino-N-(4-(N-(2,5-propyl)-pyrrolophényl))-phénylazo)-4-hydroxyméthyl-1,3-dipropyl-3H-imidazol-1-ium,
2-(4-amino-N-(4-(N-(2-methyl-5-ethyl)-pyrrolophényl))-phénylazo)-4-hydroxyméthyl-1,3-dipropyl-3H-imidazol-1-ium,
2-(4-amino-N-(4-(N-(2-méthyl-5-propyl)-pyrrolophényl))-phénylazo)-4-hydroxyméthyl-1,3-dipropyl-3H-imidazol-1-ium,
2-(4-amino-N-(4-(N-(2-méthyl-5-phényl)-pyrrolophényl))-phénylazo)-4-hydroxyméthyl-1,3-dipropyl-3H-imidazol-1-ium,
2-(4-amino-N-(4-(N-pyrrolophényl))-phénylazo)-4-hydroxyméthyl-1,3-di(2-hydroxyethyl)-3H-imidazol-1-ium,
2-(4-amino-N-(4-(N-(2,5-diméthyl)-pyrrolophényl))-phénylazo)-4-hydroxyméthyl-1,3-di(2-hydroxyethyl)-3H-imidazol-1-ium,
2-(4-amino-N-(4-(N-(2,5-diéthyl)-pyrrolophényl))-phénylazo)-4-hydroxyméthyl-1,3-di(2-hydroxyethyl)-3H-imidazol-1-ium,
2-(4-amino-N-(4-(N-(2,5-propyl)-pyrrolophényl))-phénylazo)-4-hydroxyméthyl-1,3-di(2-hydroxyethyl)-3H-imidazol-1-ium,
2-(4-amino-N-(4-(N-(2-methyl-5-ethyl)-pyrrolophényl))-phénylazo)-4-hydroxyméthyl-1,3-di(2-hydroxyethyl)-3H-imidazol-1-ium,
2-(4-amino-N-(4-(N-(2-méthyl-5-propyl)-pyrrolophényl))-phénylazo)-4-hydroxyméthyl-1,3-di(2-hydroxyethyl)-3H-imidazol-1-ium,
2-(4-amino-N-(4-(N-(2-méthyl-5-phényl)-pyrrolophényl))-phénylazo)-4-hydroxyméthyl-1,3-di(2-hydroxyethyl)-3H-imidazol-1-ium,
2-(4-amino-N-(4-(N-imidazolophényl))-phénylazo)-1,3-diméthyl-3H-imidazol-1-ium,
2-(4-amino-N-(4-(N-imidazolophényl))-phénylazo)-1,3-diéthyl-3H-imidazol-1-ium,
2-(4-amino-N-(4-(N-imidazolophényl))-phénylazo)-1,3-dipropyl-3H-imidazol-1-ium,
2-(4-amino-N-(4-(N-imidazolophényl))-phénylazo)-1,3-di(2-hydroxyethyl)-3H-imidazol-1-ium,
2-(4-amino-N-(4-(N-pyrrolophényl))-phénylazo)-1-méthyl-pyridin-1-ium,
2-(4-amino-N-(4-(N-(2,5-diméthyl)-pyrrolophényl))-phénylazo)-1-méthyl-pyridin-1-ium,
2-(4-amino-N-(4-(N-(2,5-diéthyl)-pyrrolophényl))-phénylazo)-1-méthyl-pyridin-1-ium,
2-(4-amino-N-(4-(N-(2,5-propyl)-pyrrolophényl))-phénylazo)-1-méthyl-pyrid in-1-ium,
2-(4-amino-N-(4-(N-(2-methyl-5-ethyl)-pyrrolophényl))-phénylazo)-1-méthyl-pyridin-1-ium,
2-(4-amino-N-(4-(N-(2-méthyl-5-propyl)-pyrrolophényl))-phénylazo)-1-méthyl-pyridin-1-ium,
2-(4-amino-N-(4-(N-(2-méthyl-5-phényl)-pyrrolophényl))-phénylazo)-1-méthyl-pyridin-1-ium,
2-(4-amino-N-(4-(N-pyrrolophényl))-phénylazo)-1-éthyl-pyridin-1-ium,
2-(4-amino-N-(4-(N-(2,5-diméthyl)-pyrrolophényl))-phénylazo)-1-éthyl-pyridin-1-ium,
2-(4-amino-N-(4-(N-(2,5-diéthyl)-pyrrolophényl))-phénylazo)-1-éthyl-pyridin-1-ium,
2-(4-amino-N-(4-(N-(2,5-propyl)-pyrrolophényl))-phénylazo)-1-éthyl-pyridin-1-ium,
2-(4-amino-N-(4-(N-(2-methyl-5-ethyl)-pyrrolophényl))-phénylazo)-1-éthyl-pyridin-1-ium,
2-(4-amino-N-(4-(N-(2-méthyl-5-propyl)-pyrrolophényl))-phénylazo)-1-éthyl-pyridin-1-ium,
2-(4-amino-N-(4-(N-(2-méthyl-5-phényl)-pyrrolophényl))-phénylazo)-1-éthyl-pyridin-1-ium,
2-(4-amino-N-(4-(N-pyrrolophényl))-phénylazo)-1-propyl-pyridin-1-ium,
2-(4-amino-N-(4-(N-(2,5-diméthyl)-pyrrolophényl))-phénylazo)-1-propyl-pyridin-1-ium,
2-(4-amino-N-(4-(N-(2,5-diéthyl)-pyrrolophényl))-phénylazo)-1-propyl-pyridin-1-ium,
2-(4-amino-N-(4-(N-(2,5-propyl)-pyrrolophényl))-phénylazo)-1-propyl-pyridin-1-ium,
2-(4-amino-N-(4-(N-(2-methyl-5-ethyl)-pyrrolophényl))-phénylazo)-1-propyl-pyridin-1-ium,
2-(4-amino-N-(4-(N-(2-méthyl-5-propyl)-pyrrolophényl))-phénylazo)-1-propyl-pyridin-1-ium,
2-(4-amino-N-(4-(N-(2-méthyl-5-phényl)-pyrrolophényl))-phénylazo)-1-propyl-pyridin-1-ium,
2-(4-amino-N-(4-(N-pyrrolophényl))-phénylazo)-1-(2-hydroxyethyl)-pyridin-1-ium,
2-(4-amino-N-(4-(N-(2,5-diméthyl)-pyrrolophényl))-phénylazo)-1-(2-hydroxyethyl)-pyridin-1-ium,
2-(4-amino-N-(4-(N-(2,5-diéthyl)-pyrrolophényl))-phénylazo)-1-(2-hydroxyethyl)-pyridin-1-ium,
2-(4-amino-N-(4-(N-(2,5-propyl)-pyrrolophényl))-phénylazo)-1-(2-hydroxyethyl)-pyridin-1-ium,
2-(4-amino-N-(4-(N-(2-methyl-5-ethyl)-pyrrolophényl))-phénylazo)-1-(2-hydroxyethyl)-pyridin-1-ium,
2-(4-amino-N-(4-(N-(2-méthyl-5-propyl)-pyrrolophényl))-phénylazo)-1-(2-hydroxyethyl)-pyridin-1-ium,
2-(4-amino-N-(4-(N-(2-méthyl-5-phényl)-pyrrolophényl))-phénylazo)-1-(2-hydroxyethyl)-pyridin-1-ium,
2-(4-amino-N-(4-(N-pyrrolophényl))-phénylazo)-1,3-diméthyl-pyridin-1-ium,
2-(4-amino-N-(4-(N-(2,5-diméthyl)-pyrrolophényl))-phénylazo)-1,3-diméthyl-pyridin-1-ium,
2-(4-amino-N-(4-(N-(2,5-diéthyl)-pyrrolophényl))-phénylazo)-1,3-diméthyl-pyridin-1-ium,
2-(4-amino-N-(4-(N-(2,5-propyl)-pyrrolophényl))-phénylazo)-1,3-diméthyl-pyridin-1-ium,
2-(4-amino-N-(4-(N-(2-methyl-5-ethyl)-pyrrolophényl))-phénylazo)-1,3-diméthyl-pyridin-1-ium,
2-(4-amino-N-(4-(N-(2-méthyl-5-propyl)-pyrrolophényl))-phénylazo)-1,3-diméthyl -pyridin-1-ium,
2-(4-amino-N-(4-(N-(2-méthyl-5-phényl)-pyrrolophényl))-phénylazo)-1,3-diméthyl-pyridin-1-ium,
2-(4-amino-N-(4-(N-pyrrolophényl))-phénylazo)-1-éthyl-3-méthyl-pyridin-1-ium,
2-(4-amino-N-(4-(N-(2,5-diméthyl)-pyrrolophényl))-phénylazo)-1-éthyl-3-méthyl-pyridin-1-ium,
2-(4-amino-N-(4-(N-(2,5-diéthyl)-pyrrolophényl))-phénylazo)-1-éthyl-3-méthyl-pyridin-1-ium,
2-(4-amino-N-(4-(N-(2,5-propyl)-pyrrolophényl))-phénylazo)-1-éthyl-3-méthyl-pyridin-1-ium,
2-(4-amino-N-(4-(N-(2-methyl-5-ethyl)-pyrrolophényl))-phénylazo)-1-éthyl-3-méthyl-pyridin-1-ium,
2-(4-amino-N-(4-(N-(2-méthyl-5-propyl)-pyrrolophényl))-phénylazo)-1-éthyl-3-méthyl-pyridin-1-ium,
2-(4-amino-N-(4-(N-(2-méthyl-5-phényl)-pyrrolophényl))-phénylazo)-1-éthyl-3-méthyl-pyridin-1-ium,
2-(4-amino-N-(4-(N-pyrrolophényl))-phénylazo)-1-propyl-3-méthyl-pyridin-1-ium,
2-(4-amino-N-(4-(N-(2,5-diméthyl)-pyrrolophényl))-phénylazo)-1-propyl-3-méthyl-pyridin-1-ium,
2-(4-amino-N-(4-(N-(2,5-diéthyl)-pyrrolophényl))-phénylazo)-1-propyl-3-méthyl-pyridin-1-ium,
2-(4-amino-N-(4-(N-(2,5-propyl)-pyrrolophényl))-phénylazo)-1-propyl-3-méthyl-pyridin-1-ium,
2-(4-amino-N-(4-(N-(2-methyl-5-ethyl)-pyrrolophényl))-phénylazo)-1-propyl-3-méthyl-pyridin-1-ium,
2-(4-amino-N-(4-(N-(2-méthyl-5-propyl)-pyrrolophényl))-phénylazo)-1-propyl-3-méthyl-pyridin-1-ium,
2-(4-amino-N-(4-(N-(2-méthyl-5-phényl)-pyrrolophényl))-phénylazo)-1-propyl-3-méthyl-pyridin-1-ium,
2-(4-amino-N-(4-(N-pyrrolophényl))-phénylazo)-1-(2-hydroxyethyl)-3-méthyl-pyridin-1-ium,
2-(4-amino-N-(4-(N-(2,5-diméthyl)-pyrrolophényl))-phénylazo)-1-(2-hyd roxyethyl )-3-méthyl-pyridin-1-ium,
2-(4-amino-N-(4-(N-(2,5-diéthyl)-pyrrolophényl))-phénylazo)-1-(2-hyd roxyethyl )-3-méthyl-pyridin-1-ium,
2-(4-amino-N-(4-(N-(2,5-propyl)-pyrrolophényl))-phénylazo)-1-(2-hydroxyethyl)-3-méthyl-pyridin-1-ium,
2-(4-amino-N-(4-(N-(2-methyl-5-ethyl)-pyrrolophényl))-phénylazo)-1-(2-hydroxyethyl)-3-méthyl-pyridin-1-ium,
2-(4-amino-N-(4-(N-(2-méthyl-5-propyl)-pyrrolophényl))-phénylazo)-1-(2-hydroxyethyl)-3-méthyl-pyridin-1-ium,
2-(4-amino-N-(4-(N-(2-méthyl-5-phényl)-pyrrolophényl))-phénylazo)-1-(2-hyd roxyethyl )-3-méthyl-pyridin-1-ium,
2-(4-amino-N-(4-(N-pyrrolophényl))-phénylazo)-1,4-diméthyl-pyridin-1-ium,
2-(4-amino-N-(4-(N-(2,5-diméthyl)-pyrrolophényl))-phénylazo)-1,4-diméthyl-pyridin-1-ium,
2-(4-amino-N-(4-(N-(2,5-diéthyl)-pyrrolophényl))-phénylazo)-1,4-diméthyl-pyridin-1-ium,
2-(4-amino-N-(4-(N-(2,5-propyl)-pyrrolophényl))-phénylazo)-1,4-diméthyl-pyridin-1-ium,
2-(4-amino-N-(4-(N-(2-methyl-5-ethyl)-pyrrolophényl))-phénylazo)-1,4-diméthyl-pyridin-1-ium,
2-(4-amino-N-(4-(N-(2-méthyl-5-propyl)-pyrrolophényl))-phénylazo)-1,4-diméthyl -pyridin-1-ium,
2-(4-amino-N-(4-(N-(2-méthyl-5-phényl)-pyrrolophényl))-phénylazo)-1,4-diméthyl-pyridin-1-ium,
2-(4-amino-N-(4-(N-pyrrolophényl))-phénylazo)-1-éthyl-4-méthyl-pyridin-1-ium,
2-(4-amino-N-(4-(N-(2,5-diméthyl)-pyrrolophényl))-phénylazo)-1-éthyl-4-méthyl-pyridin-1-ium,
2-(4-amino-N-(4-(N-(2,5-diéthyl)-pyrrolophényl))-phénylazo)-1-éthyl-4-méthyl-pyridin-1-ium,
2-(4-amino-N-(4-(N-(2,5-propyl)-pyrrolophényl))-phénylazo)-1-éthyl-4-méthyl-pyrid in-1-ium,
2-(4-amino-N-(4-(N-(2-methyl-5-ethyl)-pyrrolophényl))-phénylazo)-1-éthyl-4-méthyl-pyridin-1-ium,
2-(4-amino-N-(4-(N-(2-méthyl-5-propyl)-pyrrolophényl))-phénylazo)-1-éthyl-4-méthyl-pyridin-1-ium,
2-(4-amino-N-(4-(N-(2-méthyl-5-phényl)-pyrrolophényl))-phénylazo)-1-éthyl-4-méthyl-pyridin-1-ium,
2-(4-amino-N-(4-(N-pyrrolophényl))-phénylazo)-1-propyl-4-méthyl-pyridin-1-ium,
2-(4-amino-N-(4-(N-(2,5-diméthyl)-pyrrolophényl))-phénytazo)-1-propyl-4-méthyl-pyridin-1-ium,
2-(4-amino-N-(4-(N-(2,5-diéthyl)-pyrrolophényl))-phénylazo)-1-propyl-4-méthyl-pyridin-1-ium,
2-(4-amino-N-(4-(N-(2,5-propyl)-pyrrolophényl))-phénylazo)-1-propyl-4-méthyl-pyridin-1-ium,
2-(4-amino-N-(4-(N-(2-methyl-5-ethyl)-pyrrolophényl))-phénylazo)-1-propyl-4-méthyl-pyridin-1-ium,
2-(4-amino-N-(4-(N-(2-méthyl-5-propyl)-pyrrolophényl))-phénylazo)-1-propyl-4-méthyl-pyridin-1-ium,
2-(4-amino-N-(4-(N-(2-méthyl-5-phényl)-pyrrolophényl))-phénylazo)-1-propyl-4-méthyl-pyridin-1-ium,
2-(4-amino-N-(4-(N-pyrrolophényl))-phénylazo)-1-(2-hydroxyethyl)-4-méthyl-pyrid in-1-ium,
2-(4-amino-N-(4-(N-(2,5-diméthyl)-pyrrolophényl))-phénylazo)-1-(2-hydroxyethyl)-4-méthyl-pyridin-1-ium,
2-(4-amino-N-(4-(N-(2,5-diéthyl)-pyrrolophényl))-phénylazo)-1-(2-hydroxyethyl)-4-méthyl-pyridin-1-ium,
2-(4-amino-N-(4-(N-(2,5-propyl)-pyrrolophényl))-phénylazo)-1-(2-hydroxyethyl)-4-méthyl-pyridin-1-ium,
2-(4-amino-N-(4-(N-(2-methyl-5-ethyl)-pyrrolophényl))-phénylazo)-1-(2-hydroxyethyl )-4-méthyl-pyridin-1-ium,
2-(4-amino-N-(4-(N-(2-méthyl-5-propyl)-pyrrolophényl))-phénylazo)-1-(2-hydroxyethyl)-4-méthyl-pyridin-1-ium,
2-(4-amino-N-(4-(N-(2-méthyl-5-phényl)-pyrrolophényl))-phénylazo)-1-(2-hydroxyethyl)-4-méthyl-pyridin-1-ium,
2-(4-amino-N-(4-(N-pyrrolophényl))-phénylazo)-1,5-diméthyl-pyridin-1-ium,
2-(4-amino-N-(4-(N-(2,5-diméthyl)-pyrrolophényl))-phénylazo)-1,5-diméthyl-pyridin-1-ium,
2-(4-amino-N-(4-(N-(2,5-diéthyl)-pyrrolophényl))-phénylazo)-1,5-diméthyl-pyridin-1-ium,
2-(4-amino-N-(4-(N-(2,5-propyl)-pyrrolophényl))-phénylazo)-1,5-diméthyl-pyridin-1-ium,
2-(4-amino-N-(4-(N-(2-methyl-5-ethyl)-pyrrolophényl))-phénylazo)-1,5-diméthyl-pyridin-1-ium,
2-(4-amino-N-(4-(N-(2-méthyl-5-propyl)-pyrrolophényl))-phénylazo)-1,5-diméthyl -pyridin-1-ium,
2-(4-amino-N-(4-(N-(2-méthyl-5-phényl)-pyrrolophényl))-phénylazo)-1,5-diméthyl-pyridin-1-ium,
2-(4-amino-N-(4-(N-pyrrolophényl))-phénylazo)-1-éthyl-5-méthyl-pyridin-1-ium,
2-(4-amino-N-(4-(N-(2,5-diméthyl)-pyrrolophényl))-phénylazo)-1-éthyl-5-méthyl-pyridin-1-ium,
2-(4-amino-N-(4-(N-(2,5-diéthyl)-pyrrolophényl))-phénylazo)-1-éthyl-5-méthyl-pyridin-1-ium,
2-(4-amino-N-(4-(N-(2,5-propyl)-pyrrolophényl))-phénylazo)-1-éthyl-5-méthyl-pyridin-1-ium,
2-(4-amino-N-(4-(N-(2-methyl-5-ethyl)-pyrrolophényl))-phénylazo)-1-éthyl-5-méthyl-pyridin-1-ium,
2-(4-amino-N-(4-(N-(2-méthyl-5-propyl)-pyrrolophényl))-phénylazo)-1-éthyl-5-méthyl-pyridin-1-ium,
2-(4-amino-N-(4-(N-(2-méthyl-5-phényl)-pyrrolophényl))-phénylazo)-1-éthyl-5-méthyl-pyridin-1-ium,
2-(4-amino-N-(4-(N-pyrrolophényl))-phénylazo)-1-propyl-5-méthyl-pyrid in-1-ium,
2-(4-amino-N-(4-(N-(2,5-diméthyl)-pyrrolophényl))-phénylazo)-1-propyl-5-méthyl-pyridin-1-ium,
2-(4-amino-N-(4-(N-(2,5-diéthyl)-pyrrolophényl))-phénylazo)-1-propyl-5-méthyl-pyridin-1-ium,
2-(4-amino-N-(4-(N-(2,5-propyl)-pyrrolophényl))-phénylazo)-1-propyl-5-méthyl-pyridin-1-ium,
2-(4-amino-N-(4-(N-(2-methyl-5-ethyl)-pyrrolophényl))-phénylazo)-1-propyl-5-méthyl-pyridin-1-ium,
2-(4-amino-N-(4-(N-(2-méthyl-5-propyl)-pyrrolophényl))-phénylazo)-1-propyl-5-méthyl-pyridin-1-ium,
2-(4-amino-N-(4-(N-(2-méthyl-5-phényl)-pyrrolophényl))-phénylazo)-1-propyl-5-méthyl-pyridin-1-ium,
2-(4-amino-N-(4-(N-pyrrolophényl))-phénylazo)-1-(2-hydroxyethyl)-5-méthyl-pyridin-1-ium,
2-(4-amino-N-(4-(N-(2,5-diméthyl)-pyrrolophényl))-phénylazo)-1-(2-hydroxyethyl)-5-méthyl-pyridin-1-ium,
2-(4-amino-N-(4-(N-(2,5-diéthyl)-pyrrolophényl))-phénylazo)-1-(2-hydroxyethyl )-5-méthyl-pyridin-1-ium,
2-(4-amino-N-(4-(N-(2,5-propyl)-pyrrolophényl))-phénylazo)-1-(2-hydroxyethyl)-5-méthyl-pyridin-1-ium,
2-(4-amino-N-(4-(N-(2-methyl-5-ethyl)-pyrrolophényl))-phénylazo)-1-(2-hydroxyethyl)-5-méthyl-pyridin-1-ium,
2-(4-amino-N-(4-(N-(2-méthyl-5-propyl)-pyrrolophényl))-phénylazo)-1-(2-hydroxyethyl)-5-méthyl-pyridin-1-ium,
2-(4-amino-N-(4-(N-(2-méthyl-5-phényl)-pyrrolophényl))-phénylazo)-1-(2-hydroxyethyl)-5-méthyl-pyridin-1-ium;
2-(4-amino-N-(4-(N-pyrrolophényl))-phénylazo)-1,6-diméthyl-pyridin-1-ium,
2-(4-amino-N-(4-(N-(2,5-diméthyl)-pyrrolophényl))-phénylazo)-1,6-diméthyl-pyridin-1-ium,
2-(4-amino-N-(4-(N-(2,5-diéthyl)-pyrrolophényl))-phénylazo)-1,6-diméthyl-pyridin-1-ium,
2-(4-amino-N-(4-(N-(2,5-propyl)-pyrrolophényl))-phénylazo)-1,6-diméthyl-pyridin-1-ium,
2-(4-amino-N-(4-(N-(2-methyl-5-ethyl)-pyrrolophényl))-phénylazo)-1,6-diméthyl-pyridin-1-ium,
2-(4-amino-N-(4-(N-(2-méthyl-5-propyl)-pyrrolophényl))-phénylazo)-1,6-diméthyl -pyridin-1-ium,
2-(4-amino-N-(4-(N-(2-méthyl-5-phényl)-pyrrolophényl))-phénylazo)-1,6-diméthyl-pyridin-1-ium,
2-(4-amino-N-(4-(N-pyrrolophényl))-phénylazo)-1-éthyl-6-méthyl-pyridin-1-ium,
2-(4-amino-N-(4-(N-(2,5-diméthyl)-pyrrolophényl))-phénylazo)-1-éthyl-6-méthyl-pyridin-1-ium,
2-(4-amino-N-(4-(N-(2,5-diéthyl)-pyrrolophényl))-phénylazo)-1-éthyl-6-méthyl-pyridin-1-ium,
2-(4-amino-N-(4-(N-(2,5-propyl)-pyrrolophényl))-phénylazo)-1-éthyl-6-méthyl-pyridin-1-ium,
2-(4-amino-N-(4-(N-(2-methyl-5-ethyl)-pyrrolophényl))-phénylazo)-1-éthyl-6-méthyl-pyridin-1-ium,
2-(4-amino-N-(4-(N-(2-méthyl-5-propyl)-pyrrolophényl))-phénylazo)-1-éthyl-6-méthyl-pyridin-1-ium,
2-(4-amino-N-(4-(N-(2-méthyl-5-phényl)-pyrrolophényl))-phénylazo)-1-éthyl-6-méthyl-pyridin-1-ium,
2-(4-amino-N-(4-(N-pyrrolophényl))-phénylazo)-1-propyl-6-méthyl-pyridin-1-ium,
2-(4-amino-N-(4-(N-(2,5-diméthyl)-pyrrolophényl))-phénylazo)-1-propyl-6-méthyl-pyridin-1-ium,
2-(4-amino-N-(4-(N-(2,5-diéthyl)-pyrrolophényl))-phénylazo)-1-propyl-6-méthyl-pyridin-1-ium,
2-(4-amino-N-(4-(N-(2,5-propyl)-pyrrolophényl))-phénylazo)-1-propyl-6-méthyl-pyridin-1-ium,
2-(4-amino-N-(4-(N-(2-methyl-5-ethyl)-pyrrolophényl))-phénylazo)-1-propyl-6-méthyl-pyridin-1-ium,
2-(4-amino-N-(4-(N-(2-méthyl-5-propyl)-pyrrolophényl))-phénylazo)-1-propyl-6-méthyl-pyridin-1-ium,
2-(4-amino-N-(4-(N-(2-méthyl-5-phényl)-pyrrolophényl))-phénylazo)-1-propyl-6-méthyl-pyridin-1-ium,
2-(4-amino-N-(4-(N-pyrrolophényl))-phénylazo)-1-(2-hydroxyethyl)-6-méthyl-pyridin-1-ium,
2-(4-amino-N-(4-(N-(2,5-diméthyl)-pyrrolophényl))-phénylazo)-1-(2-hydroxyethyl)-6-méthyl-pyridin-1-ium,
2-(4-amino-N-(4-(N-(2,5-diéthyl)-pyrrolophényl))-phénylazo)-1-(2-hydroxyethyl)-6-méthyl-pyridin-1-ium,
2-(4-amino-N-(4-(N-(2,5-propyl)-pyrrolophényl))-phénylazo)-1-(2-hydroxyethyl )-6-méthyl-pyridin-1-ium,
2-(4-amino-N-(4-(N-(2-methyl-5-ethyl)-pyrrolophényl))-phénylazo)-1-(2-hydroxyethyl)-6-méthyl-pyridin-1-ium,
2-(4-amino-N-(4-(N-(2-méthyl-5-propyl)-pyrrolophényl))-phénylazo)-1-(2-hydroxyethyl)-6-méthyl-pyridin-1-ium,
2-(4-amino-N-(4-(N-(2-méthyl-5-phényl)-pyrrolophényl))-phénylazo)-1-(2-hydroxyethyl)-6-méthyl-pyridin-1-ium,
2-(4-amino-N-(4-(N-pyrrolophényl))-phénylazo)-1,4,6-triméthyl-pyridin-1-ium,
2-(4-amino-N-(4-(N-(2,5-diméthyl)-pyrrolophényl))-phénylazo)-1,4,6-triméthyl-pyridin-1-ium,
2-(4-amino-N-(4-(N-(2,5-diéthyl)-pyrrolophényl))-phénylazo)-1,4,6-triméthyl-pyridin-1-ium,
2-(4-amino-N-(4-(N-(2,5-propyl)-pyrrolophényl))-phénylazo)-1,4,6-triméthyl-pyridin-1-ium,
2-(4-amino-N-(4-(N-(2-methyl-5-ethyl)-pyrrolophényl))-phénylazo)-1,4,6-triméthyl-pyridin-1-ium,
2-(4-amino-N-(4-(N-(2-méthyl-5-propyl)-pyrrolophényl))-phénylazo)-1,4,6-triméthyl-pyridin-1-ium,
2-(4-amino-N-(4-(N-(2-méthyl-5-phényl)-pyrrolophényl))-phénylazo)-1,4,6-triméthyl-pyridin-1-ium,
2-(4-amino-N-(4-(N-pyrrolophényl))-phénylazo)-1-éthyl-4,6-diméthyl-pyridin-1-ium,
2-(4-amino-N-(4-(N-(2,5-diméthyl)-pyrrolophényl))-phénylazo)-1-éthyl-4,6-diméthyl-pyridin-1-ium,
2-(4-amino-N-(4-(N-(2,5-diéthyl)-pyrrolophényl))-phénylazo)-1-éthyl-4,6-diméthyl-pyridin-1-ium,
2-(4-amino-N-(4-(N-(2,5-propyl)-pyrrolophényl))-phénylazo)-1-éthyl-4,6-diméthyl-pyridin-1-ium,
2-(4-amino-N-(4-(N-(2-methyl-5-ethyl)-pyrrolophényl))-phénylazo)-1-éthyl-4,6-diméthyl-pyridin-1-ium,
2-(4-amino-N-(4-(N-(2-méthyl-5-propyl)-pyrrolophényl))-phénylazo)-1-éthyl-4,6-diméthyl-pyridin-1-ium,
2-(4-amino-N-(4-(N-(2-méthyl-5-phényl)-pyrrolophényl))-phénylazo)-1-éthyl-4,6-diméthyl-pyridin-1-ium,
2-(4-amino-N-(4-(N-pyrrolophényl))-phénylazo)-1-propyl-4,6-diméthyl-pyridin-1-ium,
2-(4-amino-N-(4-(N-(2,5-diméthyl)-pyrrolophényl))-phénylazo)-1-propyl-4,6-diméthyl-pyridin-1-ium,
2-(4-amino-N-(4-(N-(2,5-diéthyl)-pyrrolophényl))-phénylazo)-1-propyl-4,6-diméthyl-pyridin-1-ium,
2-(4-amino-N-(4-(N-(2,5-propyl)-pyrrolophényl))-phénylazo)-1-propyl-4,6-diméthyl-pyridin-1-ium,
2-(4-amino-N-(4-(N-(2-methyl-5-ethyl)-pyrrolophényl))-phénylazo)-1-propyl-4,6-diméthyl-pyridin-1-ium,
2-(4-amino-N-(4-(N-(2-méthyl-5-propyl)-pyrrolophényl))-phénylazo)-1-propyl-4,6-diméthyl-pyridin-1-ium,
2-(4-amino-N-(4-(N-(2-méthyl-5-phényl)-pyrrolophényl))-phénylazo)-1-propyl-4,6-diméthyl-pyridin-1-ium,
2-(4-amino-N-(4-(N-pyrrolophényl))-phénylazo)-1-(2-hydroxyethyl)-4,6-diméthyl-pyrid in-1-ium,
2-(4-amino-N-(4-(N-(2,5-diméthyl)-pyrrolophényl))-phénylazo)-1-(2-hydroxyethyl)-4,6-diméthyl-pyridin-1-ium,
2-(4-amino-N-(4-(N-(2,5-diéthyl)-pyrrolophényl))-phénylazo)-1-(2-hydroxyethyl)-4,6-diméthyl-pyridin-1-ium,
2-(4-amino-N-(4-(N-(2,5-propyl)-pyrrolophényl))-phénylazo)-1-(2-hydroxyethyl)-4,6-diméthyl-pyridin-1-ium,
2-(4-amino-N-(4-(N-(2-methyl-5-ethyl)-pyrrolophényl))-phénylazo)-1-(2-hydroxyethyl )-4,6-diméthyl-pyridin-1-ium,
2-(4-amino-N-(4-(N-(2-méthyl-5-propyl)-pyrrolophényl))-phénylazo)-1-(2-hydroxyethyl )-4,6-diméthyl-pyridin-1-ium,
2-(4-amino-N-(4-(N-(2-méthyl-5-phényl)-pyrrolophényl))-phénylazo)-1-(2-hydroxyethyl)-4,6-diméthyl-pyridin-1-ium,
2-(4-amino-N-(4-(N-pyrrolophényl))-phénylazo)-1-méthyl-2,4-dichloro-pyridin-1-ium,
2-(4-amino-N-(4-(N-(2,5-diméthyl)-pyrrolophényl))-phénylazo)-1-méthyl-2,4-dichloro-pyridin-1-ium,
2-(4-amino-N-(4-(N-(2,5-diéthyl)-pyrrolophényl))-phénylazo)-1-méthyl-2,4-dichloro-pyridin-1-ium,
2-(4-amino-N-(4-(N-(2,5-propyl)-pyrrolophényl))-phénylazo)-1-méthyl-2,4-dichloro-pyridin-1-ium,
2-(4-amino-N-(4-(N-(2-methyl-5-ethyl)-pyrrolophényl))-phénylazo)-1-méthyl-2,4-dichloro-pyridin-1-ium,
2-(4-amino-N-(4-(N-(2-méthyl-5-propyl)-pyrrolophényl))-phénylazo)-1-méthyl-2,4-dichloro-pyridin-1-ium,
2-(4-amino-N-(4-(N-(2-méthyl-5-phényl)-pyrrolophényl))-phénylazo)-1-méthyl-2,4-dichloro-pyridin-1-ium,
2-(4-amino-N-(4-(N-pyrrolophényl))-phénylazo)-1-éthyl-2,4-dichloro-pyridin-1-ium,
2-(4-amino-N-(4-(N-(2,5-diméthyl)-pyrrolophényl))-phénylazo)-1-éthyl-2,4-dichloro-pyridin-1-ium,
2-(4-amino-N-(4-(N-(2,5-diéthyl)-pyrrolophényl))-phénylazo)-1-éthyl-2,4-dichloro-pyridin-1-ium,
2-(4-amino-N-(4-(N-(2,5-propyl)-pyrrolophényl))-phénylazo)-1-éthyl-2,4-dichloro-pyridin-1-ium,
2-(4-amino-N-(4-(N-(2-methyl-5-ethyl)-pyrrolophényl))-phénylazo)-1-éthyl-2,4-dichloro-pyridin-1-ium,
2-(4-amino-N-(4-(N-(2-méthyl-5-propyl)-pyrrolophényl))-phénylazo)-1-éthyl-2,4-dichloro-pyridin-1-ium,
2-(4-amino-N-(4-(N-(2-méthyl-5-phényl)-pyrrolophényl))-phénylazo)-1-éthyl-2,4-dichloro-pyridin-1-ium,
2-(4-amino-N-(4-(N-pyrrolophényl))-phénylazo)-1-propyl-2,4-dichloro-pyridin-1-ium,
2-(4-amino-N-(4-(N-(2,5-diméthyl)-pyrrolophényl))-phénylazo)-1-propyl-2,4-dichloro-pyridin-1-ium,
2-(4-amino-N-(4-(N-(2,5-diéthyl)-pyrrolophényl))-phénylazo)-1-propyl-2,4-dichloro-pyridin-1-ium,
2-(4-amino-N-(4-(N-(2,5-propyl)-pyrrolophényl))-phénylazo)-1-propyl-2,4-dichloro-pyridin-1-ium,
2-(4-amino-N-(4-(N-(2-methyl-5-ethyl)-pyrrolophényl))-phénylazo)-1-propyl-2,4-dichloro-pyridin-1-ium,
2-(4-amino-N-(4-(N-(2-méthyl-5-propyl)-pyrrolophényl))-phénylazo)-1-propyl-2,4-dichloro-pyridin-1-ium,
2-(4-amino-N-(4-(N-(2-méthyl-5-phényl)-pyrrolophényl))-phénylazo)-1-propyl-2,4-dichloro-pyridin-1-ium,
2-(4-amino-N-(4-(N-pyrrolophényl))-phénylazo)-1-(2-hydroxyethyl)-2,4-dichloro-pyridin-1-ium,
2-(4-amino-N-(4-(N-(2,5-diméthyl)-pyrrolophényl))-phénylazo)-1-(2-hydroxyethyl)-2,4-dichloro-pyridin-1-ium,
2-(4-amino-N-(4-(N-(2,5-diéthyl)-pyrrolophényl))-phénylazo)-1-(2-hydroxyethyl)-2,4-dichloro-pyridin-1-ium,
2-(4-amino-N-(4-(N-(2,5-propyl)-pyrrolophényl))-phénylazo)-1-(2-hydroxyethyl)-2,4-dichloro-pyridin-1-ium,
2-(4-amino-N-(4-(N-(2-methyl-5-ethyl)-pyrrolophényl))-phénylazo)-1-(2-hydroxyethyl)-2,4-dichloro-pyridin-1-ium,
2-(4-amino-N-(4-(N-(2-méthyl-5-propyl)-pyrrolophényl))-phénylazo)-1-(2-hydroxyethyl)-2,4-dichloro-pyridin-1-ium,
2-(4-amino-N-(4-(N-(2-méthyl-5-phényl)-pyrrolophényl))-phénylazo)-1-(2-hydroxyethyl)-2,4-dichloro-pyridin-1-ium,
2-(4-amino-N-(4-(N-pyrrolophényl))-phénylazo)-1-méthyl-5-chloro-pyridin-1-ium,
2-(4-amino-N-(4-(N-(2,5-diméthyl)-pyrrolophényl))-phénylazo)-1-méthyl-5-chloro-pyridin-1-ium,
2-(4-amino-N-(4-(N-(2,5-diéthyl)-pyrrolophényl))-phénylazo)-1-méthyl-5-chloro-pyridin-1-ium,
2-(4-amino-N-(4-(N-(2,5-propyl)-pyrrolophényl))-phénylazo)-1-méthyl-5-chloro-pyridin-1-ium,
2-(4-amino-N-(4-(N-(2-methyl-5-ethyl)-pyrrolophényl))-phénylazo)-1-méthyl-5-chloro-pyridin-1-ium,
2-(4-amino-N-(4-(N-(2-méthyl-5-propyl)-pyrrolophényl))-phénylazo)-1-méthyl-5-chloro-pyridin-1-ium,
2-(4-amino-N-(4-(N-(2-méthyl-5-phényl)-pyrrolophényl))-phénylazo)-1-méthyl-5-chloro-pyridin-1-ium,
2-(4-amino-N-(4-(N-pyrrolophényl))-phénylazo)-1-éthyl-5-chloro-pyridin-1-ium,
2-(4-amino-N-(4-(N-(2,5-diméthyl)-pyrrolophényl))-phénylazo)-1-éthyl-5-chloro-pyridin-1-ium,
2-(4-amino-N-(4-(N-(2,5-diéthyl)-pyrrolophényl))-phénylazo)-1-éthyl-5-chloro-pyridin-1-ium,
2-(4-amino-N-(4-(N-(2,5-propyl)-pyrrolophényl))-phénylazo)-1-éthyl-5-chloro-pyridin-1-ium,
2-(4-amino-N-(4-(N-(2-methyl-5-ethyl)-pyrrolophényl))-phénylazo)-1-éthyl-5-chloro-pyridin-1-ium,
2-(4-amino-N-(4-(N-(2-méthyl-5-propyl)-pyrrolophényl))-phénylazo)-1-éthyl-5-chloro-pyridin-1-ium,
2-(4-amino-N-(4-(N-(2-méthyl-5-phényl)-pyrrolophényl))-phénylazo)-1-éthyl-5-chloro-pyridin-1-ium,
2-(4-amino-N-(4-(N-pyrrolophényl))-phénylazo)-1-propyl-5-chloro-pyridin-1-ium,
2-(4-amino-N-(4-(N-(2,5-diméthyl)-pyrrolophényl))-phénylazo)-1-propyl-5-chloro-pyridin-1-ium,
2-(4-amino-N-(4-(N-(2,5-diéthyl)-pyrrolophényl))-phénylazo)-1-propyl-5-chloro-pyridin-1-ium,
2-(4-amino-N-(4-(N-(2,5-propyl)-pyrrolophényl))-phénylazo)-1-propyl-5-chloro-pyridin-1-ium,
2-(4-amino-N-(4-(N-(2-methyl-5-ethyl)-pyrrolophényl))-phénylazo)-1-propyl-5-chloro-pyridin-1-ium,
2-(4-amino-N-(4-(N-(2-méthyl-5-propyl)-pyrrolophényl))-phénylazo)-1-propyl-5-chloro-pyridin-1-ium,
2-(4-amino-N-(4-(N-(2-méthyl-5-phényl)-pyrrolophényl))-phénylazo)-1-propyl-5-chloro-pyridin-1-ium,
2-(4-amino-N-(4-(N-pyrrolophényl))-phénylazo)-1-(2-hydroxyethyl)-5-chloro-pyridin-1-ium,
2-(4-amino-N-(4-(N-(2,5-diméthyl)-pyrrolophényl))-phénylazo)-1-(2-hydroxyethyl)-5-chloro-pyridin-1-ium,
2-(4-amino-N-(4-(N-(2,5-diéthyl)-pyrrolophényl))-phénylazo)-1-(2-hydroxyethyl)-5-chloro-pyridin-1-ium,
2-(4-amino-N-(4-(N-(2,5-propyl)-pyrrolophényl))-phénylazo)-1-(2-hydroxyethyl)-5-chloro-pyridin-1-ium,
2-(4-amino-N-(4-(N-(2-methyl-5-ethyl)-pyrrolophényl))-phénylazo)-1-(2-hydroxyethyl)-5-chloro-pyridin-1-ium,
2-(4-amino-N-(4-(N-(2-méthyl-5-propyl)-pyrrolophényl))-phénylazo)-1-(2-hydroxyethyl)-5-chloro-pyridin-1-ium,
2-(4-amino-N-(4-(N-(2-méthyl-5-phényl)-pyrrolophényl))-phénylazo)-1-(2-hyd roxyethyl )-5-chloro-pyridin-1-ium,
2-(4-amino-N-(4-(N-pyrrolophényl))-phénylazo)-1-méthyl-3-hydroxy-pyridin-1-ium,
2-(4-amino-N-(4-(N-(2,5-diméthyl)-pyrrolophényl))-phénylazo)-1-méthyl-3-hydroxy-pyridin-1-ium,
2-(4-amino-N-(4-(N-(2,5-diéthyl)-pyrrolophényl))-phénylazo)-1-méthyl-3-hydroxy-pyridin-1-ium,
2-(4-amino-N-(4-(N-(2,5-propyl)-pyrrolophényl))-phénylazo)-1-méthyl-3-hydroxy-pyridin-1-ium,
2-(4-amino-N-(4-(N-(2-methyl-5-ethyl)-pyrrolophényl))-phénylazo)-1-méthyl-3-hydroxy-pyridin-1-ium,
2-(4-amino-N-(4-(N-(2-méthyl-5-propyl)-pyrrolophényl))-phénylazo)-1-méthyl-3-hydroxy-pyridin-1-ium,
2-(4-amino-N-(4-(N-(2-méthyl-5-phényl)-pyrrolophényl))-phénylazo)-1-méthyl-3-hydroxy-pyridin-1-ium,
2-(4-amino-N-(4-(N-pyrrolophényl))-phénylazo)-1-éthyl-3-hydroxy-pyridin-1-ium,
2-(4-amino-N-(4-(N-(2,5-diméthyl)-pyrrolophényl))-phénylazo)-1-éthyl-3-hydroxy-pyridin-1-ium,
2-(4-amino-N-(4-(N-(2,5-diéthyl)-pyrrolophényl))-phénylazo)-1-éthyl-3-hydroxy-pyridin-1-ium,
2-(4-amino-N-(4-(N-(2,5-propyl)-pyrrolophényl))-phénylazo)-1-éthyl-3-hydroxy-pyridin-1-ium,
2-(4-amino-N-(4-(N-(2-methyl-5-ethyl)-pyrrolophényl))-phénylazo)-1-éthyl-3-hydroxy-pyridin-1-ium,
2-(4-amino-N-(4-(N-(2-méthyl-5-propyl)-pyrrolophényl))-phénylazo)-1-éthyl-3-hydroxy-pyridin-1-ium,
2-(4-amino-N-(4-(N-(2-méthyl-5-phényl)-pyrrolophényl))-phénylazo)-1-éthyl-3-hydroxy-pyridin-1-ium,
2-(4-amino-N-(4-(N-pyrrolophényl))-phénylazo)-1-propyl-3-hydroxy-pyridin-1-ium,
2-(4-amino-N-(4-(N-(2,5-diméthyl)-pyrrolophényl))-phénylazo)-1-propyl-3-hydroxy-pyridin-1-ium,
2-(4-amino-N-(4-(N-(2,5-diéthyl)-pyrrolophényl))-phénylazo)-1-propyl-3-hydroxy-pyridin-1-ium,
2-(4-amino-N-(4-(N-(2,5-propyl)-pyrrolophényl))-phénylazo)-1-propyl-3-hydroxy-pyridin-1-ium,
2-(4-amino-N-(4-(N-(2-methyl-5-ethyl)-pyrrolophényl))-phénylazo)-1-propyl-3-hydroxy-pyridin-1-ium,
2-(4-amino-N-(4-(N-(2-méthyl-5-propyl)-pyrrolophényl))-phénylazo)-1-propyl-3-hydroxy-pyridin-1-ium,
2-(4-amino-N-(4-(N-(2-méthyl-5-phényl)-pyrrolophényl))-phénylazo)-1-propyl-3-hydroxy-pyridin-1-ium,
2-(4-amino-N-(4-(N-pyrrolophényl))-phénylazo)-1-(2-hydroxyethyl )-3-hydroxy-pyrid in-1-ium,
2-(4-amino-N-(4-(N-(2,5-diméthyl)-pyrrolophényl))-phénylazo)-1-(2-hydroxyethyl)-3-hydroxy-pyridin-1-ium,
2-(4-amino-N-(4-(N-(2,5-diéthyl)-pyrrolophényl))-phénylazo)-1-(2-hydroxyethyl)-3-hydroxy-pyridin-1-ium,
2-(4-amino-N-(4-(N-(2,5-propyl)-pyrrolophényl))-phénylazo)-1-(2-hydroxyethyl)-3-hydroxy-pyridin-1-ium,
2-(4-amino-N-(4-(N-(2-methyl-5-ethyl)-pyrrolophényl))-phénylazo)-1-(2-hydroxyethyl)-3-hydroxy-pyridin-1-ium,
2-(4-amino-N-(4-(N-(2-méthyl-5-propyl)-pyrrolophényl))-phénylazo)-1-(2-hydroxyethyl)-3-hydroxy-pyridin-1-ium,
2-(4-amino-N-(4-(N-(2-méthyl-5-phényl)-pyrrolophényl))-phénylazo)-1-(2-hydroxyethyl)-3-hydroxy-pyridin-1-ium,
2-(4-amino-N-(4-(N-imidazolophényl))-phénylazo)-1-méthyl-pyridin-1-ium,
2-(4-amino-N-(4-(N-imidazolophényl))-phénylazo)-1-éthyl-pyridin-1-ium,
2-(4-amino-N-(4-(N-imidazolophényl))-phénylazo)-1-propyl-pyridin-1-ium,
2-(4-amino-N-(4-(N-imidazolophényl))-phénylazo)-1-(2-hydroxyethyl)-pyridin-1-ium,
2-(4-amino-N-(4-(N-imidazolophényl))-phénylazo)-1,3-diméthyl-pyridin-1-ium,
2-(4-amino-N-(4-(N-imidazolophényl))-phénylazo)-1,4-diméthyl-pyridin-1-ium,
2-(4-amino-N-(4-(N-imidazolophényl))-phénylazo)-1,5-diméthyl-pyridin-1-ium,
2-(4-amino-N-(4-(N-imidazolophényl))-phénylazo)-1,6-diméthyl-pyridin-1-ium,
2-(4-amino-N-(4-(N-imidazolophényl))-phénylazo)-1,4,6-triméthyl-pyridin-1-ium,
2-(4-amino-N-(4-(N-imidazolophényl))-phénylazo)-1,6-diméthyl-pyridin-1-ium,
2-(4-amino-N-(4-(N-imidazolophényl))-phénylazo)-1-méthyl-2,4-dichloro-pyridin-1-ium,
2-(4-amino-N-(4-(N-imidazolophényl))-phénylazo)-1-méthyl-5-chloro-pyridin-1-ium,
2-(4-amino-N-(4-(N-imidazolophényl))-phénylazo)-1-éthyl-3-méthyl-pyridin-1-ium,
2-(4-amino-N-(4-(N-imidazolophényl))-phénylazo)-1-éthyl-4-méthyl-pyridin-1-ium,
2-(4-amino-N-(4-(N-imidazolophényl))-phénylazo)-1-éthyl-5-méthyl-pyridin-1-ium,
2-(4-amino-N-(4-(N-imidazolophényl))-phénylazo)-1-éthyl-6-méthyl-pyridin-1-ium,
2-(4-amino-N-(4-(N-imidazolophényl))-phénylazo)-1-éthyl-4,6-diméthyl-pyridin-1-ium,
2-(4-amino-N-(4-(N-imidazolophényl))-phénylazo)-1-éthyl-6-méthyl-pyridin-1-ium,
2-(4-amino-N-(4-(N-imidazolophényl))-phénylazo)-1-éthyl-2,4-dichloro-pyridin-1-ium,
2-(4-amino-N-(4-(N-imidazolophényl))-phénylazo)-1-éthyl-5-chloro-pyridin-1-ium,
2-(4-amino-N-(4-(N-imidazolophényl))-phénylazo)-1-propyl-3-méthyl-pyridin-1-ium,
2-(4-amino-N-(4-(N-imidazolophényl))-phénylazo)-1-propyl-4-méthyl-pyridin-1-ium,
2-(4-amino-N-(4-(N-imidazolophényl))-phénylazo)-1-propyl-5-méthyl-pyridin-1-ium,
2-(4-amino-N-(4-(N-imidazolophényl))-phénylazo)-1-propyl-6-méthyl-pyridin-1-ium,
2-(4-amino-N-(4-(N-imidazolophényl))-phénylazo)-1-propyl-4,6-diméthyl-pyridin-1-ium,
2-(4-amino-N-(4-(N-imidazolophényl))-phénylazo)-1-propyl-6-méthyl-pyridin-1-ium,
2-(4-amino-N-(4-(N-imidazolophényl))-phénylazo)-1-propyl-2,4-dichloro-pyridin-1-ium,
2-(4-amino-N-(4-(N-imidazolophényl))-phénylazo)-1-propyl-5-chloro-pyridin-1-ium,
2-(4-amino-N-(4-(N-imidazolophényl))-phénylazo)-1-(2-hydroxyethyl)-3-méthyl-pyridin-1-ium,
2-(4-amino-N-(4-(N-imidazolophényl))-phénylazo)-1-(2-hydroxyethyl)-4-méthyl-pyridin-1-ium,
2-(4-amino-N-(4-(N-imidazolophényl))-phénylazo)-1-(2-hydroxyethyl)-5-méthyl-pyridin-1-ium,
2-(4-amino-N-(4-(N-imidazolophényl))-phénylazo)-1-(2-hydroxyethyl)-6-méthyl-pyridin-1-ium,
2-(4-amino-N-(4-(N-imidazolophényl))-phénylazo)-1-(2-hydroxyethyl)-4,6-diméthyl-pyridin-1-ium,
2-(4-amino-N-(4-(N-imidazolophényl))-phénylazo)-1-(2-hydroxyethyl)-6-méthyl-pyridin-1-ium,
2-(4-amino-N-(4-(N-imidazolophényl))-phénylazo)-1-(2-hydroxyethyl)-2,4-dichloro-pyridin-1-ium,
2-(4-amino-N-(4-(N-imidazolophényl))-phénylazo)-1-(2-hydroxyethyl)-5-chloro-pyridin-1-ium,
et plus particulièrement selon l'invention, on préfère les colorants de formule (I) suivants :
2-(4-amino-N-(4-(N-pyrrolophényl))-phénylazo)-1,3-diméthyl-3H-imidazol-1-ium,
2-(4-amino-N-(4-(N-(2,5-diméthyl)-pyrrolophényl))-phénylazo)-1,3-diméthyl-3H-imidazol-1-ium,
2-(4-amino-N-(4-(N-(2-méthyl-5-propyl)-pyrrolophényl))-phénylazo)-1,3-diméthyl-3H-imidazol-1-ium,
2-(4-amino-N-(4-(N-(2-méthyl-5-phényl)-pyrrolophényl))-phénylazo)-1,3-diméthyl-3H-imidazol-1-ium,
2-(4-amino-N-(4-(N-[2-méthyl-3-carboxyéthyl-5-(4-chlorophényl)-pyrrolophényl)]-phénylazo)-1,3-diméthy)-3H-imidazol-1-ium.
chacun de ces colorants étant associé à un anion X- défini ci-dessus.

La concentration en colorant monoazoïque monocationique de formule (I) peut varier d'environ 0,001 à 5% en poids par rapport au poids total de la composition tinctoriale, et de préférence d'environ 0,05 à 2%.

La composition tinctoriale conforme à l'invention peut en outre contenir des colorants directs différents de ceux de formule (I), ces colorants pouvant notamment être choisis parmi les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs méthiniques, les colorants directs tétraazapentaméthiniques, les colorants directs triarylméthaniques, les colorants directs indoaminiques et les colorants directs naturels.

Parmi les colorants directs benzéniques, on peut citer notamment les composés suivants:
- 1,4-diamino-2-nitrobenzène
- 1-amino-2 nitro-4-β- hydroxyéthylaminobenzène
- 1-amino-2 nitro-4-bis(β-hydroxyéthyl)-aminobenzène
- 1 ,4-bis(β-hydroxyéthylamino )-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)((3-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-diamino-4-nitrobenzène
- 1-amino-2-β-hydroxyéthylamino-5-nitrobenzène
- 1,2-bis-(β-hydroxyéthylamino )-4-nitrobenzène
- 1-amino-2-tris-(hydroxyméthyl)-méthylamino-5-nitrobenzène
- 1-hydroxy-2-amino-5-nitrobenzène
- 1-hydroxy-2-amino-4-nitrobenzène
- 1-hydroxy-3-nitro-4-aminobenzène
- 1-hydroxy-2-amino-4,6-dinitrobenzène
- 1-β-hydroxyéthyloxy-2-β-hydroxyéthylamino-5-nitrobenzène
- 1-méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-hydroxy-2-chloro-6-éthylamino-4-nitrobenzène
- 1-hydroxy-2-chloro-6-amino-4-nitrobenzène
- 1-hydroxy-6-bis-(β-hydroxyéthyl)-amino-3-nitrobenzène
- 1-β-hydroxyéthylamino-2-nitrobenzène
- 1-hydroxy-4-β-hydroxyéthylamino-3-nitrobenzène.

Parmi les colorants directs azoïques, on peut citer les colorants azoiques cationiques décrits dans les demandes de brevets WO 95/15144, WO-95/01772 et EP-714954 dont le contenu fait partie intégrante de l'invention.
Parmi ces composés on peut tout particulièrement citer les colorants suivants:
- chlorure de 1,3-diméthyl-2-[[4-(diméthylamino)phényl]azo]-1 H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.

On peut également citer parmi les colorants directs azoïques les colorants suivants, décrits dans le **COLOUR INDEX INTERNATIONAL** 3^{e} édition :
- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

On peut aussi citer le 1-(4'-aminodiphénylazo)-2-méthyl-4bis-((3-hydroxyéthyl) aminobenzène et l'acide 4-hydroxy-3-(2-méthoxyphénylazo)-1-naphtalène sulfonique.

Parmi les colorants directs quinoniques on peut citer les colorants suivants :
- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99
ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1 ,4-diaminoanthraq uinone
- 2-Aminoéthylaminoanthraquinone
- 1,4-bis-(β,γ-dihydroxypropylamino)-anthraquinone.

Parmi les colorants aziniques on peut citer les composés suivants :
- Basic Blue 17
- Basic Red 2.

Parmi les colorants tétraazapentaméthiniques, on peut citer les composés suivants :

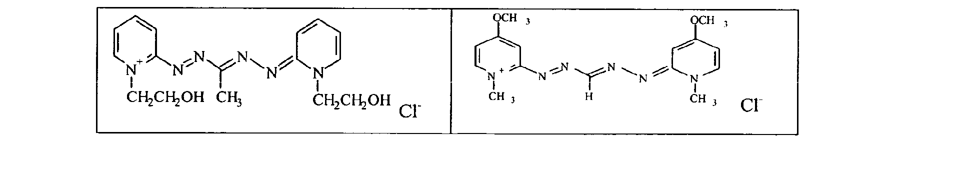

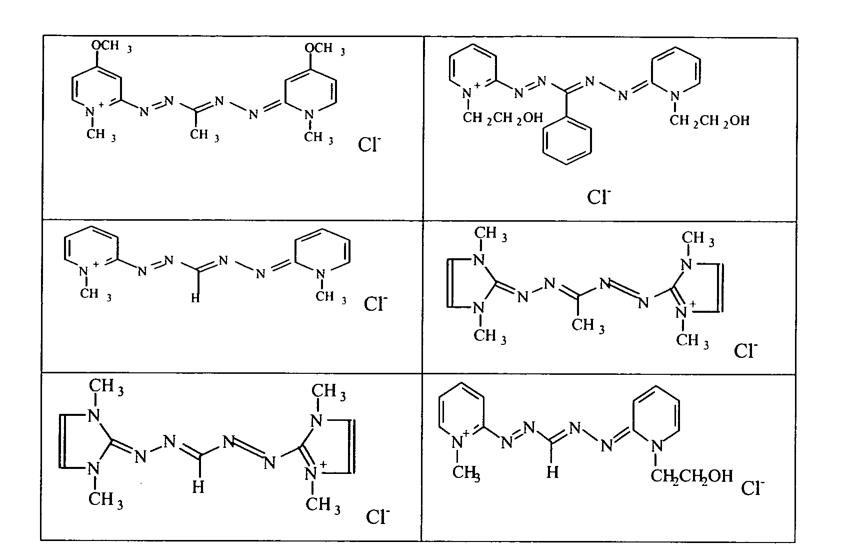

Parmi les colorants triarylméthaniques, on peut citer les composés suivants :
- Basic Green 1
- Acid blue 9
- Basic Violet 3
- Basic Violet 14
- Basic Blue 7
- Acid Violet 49
- Basic Blue 26
- Acid Blue 7

Parmi les colorants indoaminiques, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs additionnels représentent de préférence de 0,001 à 20% en poids environ du poids total de la composition et encore plus préférentiellement de 0,005 à 10% en poids environ.

La composition de l'invention peut en outre comprendre un agent oxydant. Cet agent oxydant peut être n'importe quel agent oxydant utilisé de façon classique pour la décoloration des fibres kératiniques humaines. L'agent oxydant est choisi de préférence parmi le peroxyde d'hydrogène, le peroxyde d'urée, les bromates de métaux alcalins, les persels tels que les perborates et les persulfates, les peracides et les enzymes parmi lesquelles on peut citer les peroxydases, les oxydo-réductases à 2 électrons telles que les uricases et les oxygénases à 4 électrons comme les laccases. L'utilisation du peroxyde d'hydrogène est particulièrement préférée.

Lorsque la composition selon l'invention est destinée à une teinture d'oxydation classique, elle comprend en outre une base d'oxydation. Cette base d'oxydation peut être choisie parmi les bases d'oxydation classiquement utilisées en teinture d'oxydation, par exemple les paraphénylènediamines, les bis-phénylalkylènediamines, les para-aminophénols, les ortho-aminophénols et les bases hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4 aminophenyl pyrrolidine, le 2 thiényl paraphénylène diamine, le 2-β hydroxyéthylamino 5-amino toluène et leurs sels d'addition avec un acide. Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.
Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.
Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.
Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.
Parmi les bases hétérocycliques, on peut citer à titre d'exemple, les dérivés pyridiniques, les dérivés pyrimidiniques et les dérivés pyrazoliques.
Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.
Parmi les dérivés pyrimidiniques, on peut citer les composés décrits par exemple dans les brevets DE 2 359 399 ; JP 88-169 571 ; JP 05 163 124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine; le 3-amino pyrazolo-[1,5-a]-pyrimidin-7-ol ; le 3-amino pyrazolo-[1,5-a)-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.
Parmi les dérivés pyrazoliques, on peut citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La composition selon l'invention peut contenir en outre un ou plusieurs coupleurs conventionnellement utilisés pour la teinture d'oxydation classique de fibres kératiniques humaines. Parmi ces coupleurs, on peut notamment citer les métaphénylènediamines, les méta-aminophénols, les métadiphénols, les coupleurs naphtaléniques et les coupleurs hétérocycliques.
A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.
Dans la composition de la présente invention, le ou les coupleurs sont en général présents en quantité comprise entre 0,001 et 10 % en poids environ du poids total de la composition tinctoriale et plus préférentiellement de 0,005 à 6 %. La ou les bases d'oxydation sont présentes en quantité de préférence comprise entre 0,001 à 10 % en poids environ du poids total de la composition tinctoriale, et plus préférentiellement de 0,005 à 6 %.

D'une manière générale, les sels d'addition avec un acide utilisables dans le cadre des compositions tinctoriales de l'invention pour les bases d'oxydation et les coupleurs sont notamment choisis parmi les chlorhydrates, les bromhydrates, les sulfates, les citrates, les succinates, les tartrates, les lactates, les tosylates, les benzènesulfonates, les phosphates et les acétates.

Le milieu cosmétiquement acceptable pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.
Les solvants peuvent être présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.
Ces adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. II peut être ajusté à la valeur désirée au moyen d'agents acidifiants ou alcalinisants habituellement utilisés en teinture des fibres kératiniques ou bien encore à l'aide de systèmes tampons classiques.
Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.
Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (VI) suivante : 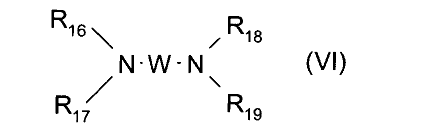 dans laquelle W est un reste propylène éventuellement substitué par un groupement hydroxyle ou un radical alkyle en C₁-C₄ ; R₁₆, R₁₇, R₁₈ et R₁₉, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques humaines, et notamment des cheveux.

L'invention a aussi pour objet un procédé de teinture directe qui comprend l'application d'une composition tinctoriale comprenant un colorant de formule (I) telle que définie précédemment, sur les fibres kératiniques humaines. Après un temps de pause, les fibres sont rincées laissant apparaître des fibres colorées.
L'application sur les fibres de la composition tinctoriale comprenant le colorant monoazoïque monocationique de formule (1) peut être mise en oeuvre en présence d'agent oxydant ce qui provoque la décoloration de la fibre (le procédé de teinture constitue alors un procédé de teinture directe éclaircissante). Cet agent oxydant peut être ajouté à la composition contenant le colorant de formule (I) au moment de l'emploi ou directement sur la fibre.

L'invention a aussi pour objet un procédé de teinture d'oxydation qui comprend l'application sur les fibres kératiniques humaines d'une composition tinctoriale qui comprend un colorant de formule (I), au moins une base d'oxydation et optionnellement au moins un coupleur, en présence d'un agent oxydant.
La base d'oxydation, le coupleur et l'agent oxydant sont tels que définis précédemment.
La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée sur les fibres simultanément ou séquentiellement à la composition tinctoriale.
Dans le cas de la teinture d'oxydation ou de la teinture directe éclaircissante , la composition tinctoriale est mélangée, de préférence au moment de l'emploi, à une composition contenant, dans un milieu cosmétiquement acceptable, au moins un agent oxydant, cet agent oxydant étant présent en une quantité suffisante pour développer une coloration. Le mélange obtenu est ensuite appliqué sur les fibres. Après un temps de pause de 3 à 50 minutes environ, de préférence 5 à 30 minutes environ, les fibres sont rincées, lavées au shampooing, rincées à nouveau puis séchées.
La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques humaines varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques humaines et tels que définis précédemment.
La composition qui est finalement appliquée sur les fibres peut se présenter sous des formes diverses, telles que sous forme de liquides, de crèmes, de gels ou sous toute autre forme appropriée pour réaliser une teinture des fibres kératiniques humaines, et notamment des cheveux.

Un autre objet de l'invention est un dispositif à plusieurs compartiments ou "kit" de teinture dans lequel un premier compartiment renferme la composition tinctoriale de l'invention et un deuxième compartiment renferme la composition oxydante. Ce dispositif peut être équipé d'un moyen permettant de délivrer sur les cheveux le mélange souhaité, tel que les dispositifs décrits dans le brevet FR-2 586 913 au nom de la demanderesse.

Enfin l'invention a également pour objet l'utilisation des colorants monoazoïques monocationiques de formule (I) à titre de colorants directs dans une, ou pour la préparation d'une composition de teinture des fibres kératiniques humaines, et plus particulièrement des cheveux.
Enfin l'invention a aussi pour objet les colorants monoazoïques monocationiques de formule (I) telle que définie précédemment et pour laquelle quand W₅ désigne imidazole, et que W₁ désigne benzimidazole, W₃ est différent de l'hydrogène si W₄ désigne phényle, (benzimidazole c'est à dire que Z₁= NR₂ R₁=R₂=CH₃, Z₂=CR₄, Z₃=CR₁₃ et R₄ avec R₁₃ forment ensemble un radical phényle).
Elle a aussi pour objet les colorants monoazoïques monocationiques de formule (I) telle que définie précédemment et pour laquelle :
- W₁ représente un hétérocycle aromatique cationique à 5 ou 6 chaînons de formules (II) et (III) suivantes : 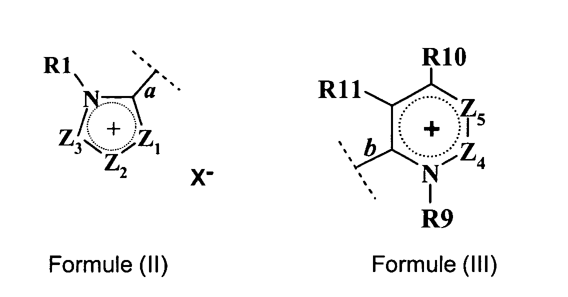
dans lesquelles :
- Z₁ représente un atome d'oxygène, un atome de soufre, un radical NR₂, ou un radical CR₃,
- Z₂ représente un atome d'azote ou un radical CR₄,
- Z₃ représente un radical NR₁₂ ou un radical CR₁₃,
- Z₄ représente un atome d'azote ou un radical CR₁₄,
- Z₅ représente un atome d'azote ou un radical CR₁₅,
   Etant entendu que les formules (II) et (III) ne contiennent pas plus de deux hétéroatomes contigus ;
- La liaison a relie le cycle cationique à 5 chaînons de formule (II) à !a fonction azoïque de la formule (1).
- la liaison b du cycle cationique à 6 chaînons de la formule (III) est reliée à la fonction azoïque de la formule (I).
- X- est un anion organique ou minéral.
- W₂ et W₄ (formule I) représentent, identiques ou différents, un groupe divalent aromatique carboné ou pyridinique de formules (IV) ou (V) suivantes : 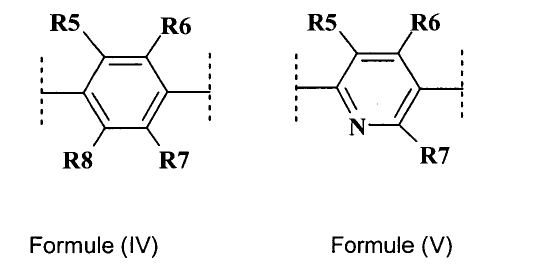
- W₃ représente un atome d'hydrogène ou un radical alkyle en C₁-C₆ pouvant éventuellement être substitué par un ou plusieurs radicaux hydroxy, un ou plusieurs radicaux alcoxy, un ou plusieurs radicaux amino, un ou plusieurs radicaux monoalkylamino en C₁-C₄, un ou plusieurs radicaux dialkylamino en C₁-C₄.
- W₅ représente un radical hétéroaromatique azoté à 5 chaînons relié à W₄ par l'atome d'azote du cycle dudit radical hétéroaromatique, ce radical héréroaromatique étant choisi parmi les radicaux pyrazolyle, pyrrolyle, imidazolyle, triazolyle, thiadiazolyle, chacun de ces radicaux hétéroaromatiques pouvant être substitué par un ou plusieurs atomes d'hydrogène, de chlore ou de fluor, ou par un ou plusieurs radicaux alkyle en C₁-C₆, éventuellement substitués par un ou plusieurs radicaux hydroxy, alcoxy en C₁-C₄, (poly)-hydroxyalcoxy en C₂-C₄, amino, (di)alkylamino en C₁-C₄, (poly)hydroxyalkylamino en C₂-C₄, carboxyle, sulfonyle, alcoxycarbonyle en C₁-C₄, thioether en C₁-C₄ ; ou par un ou plusieurs radicaux phényle éventuellement substitués par un ou plusieurs radicaux choisis parmi les radicaux hydroxy, alcoxy en C₁-C₂, amino, (di)-alkylamino en C₁-C₂, carboxy, sulfonyle, alkyle en C₁-C₄, halogène, thioéther en C₁-C₂,
- R₁, R₂, R₉, R₁₂ représentent, identiques ou différents, un radical phényle éventuellement substitué ou un radical alkyle en C₁-C₈, éventuellement substitué par un ou plusieurs radicaux choisis dans le groupe formé par les radicaux hydroxy, alcoxy en C₁-C₂, (poly)-hydroxyalcoxy en C₂-C₄, amino, (di)alkylamino en C₁-C₂,
- R₅, R₆, R₇ et R₈ représentent, identiques ou différents, un atome d'hydrogène ; un atome de chlore ; un atome de brome ; une chaîne hydrocarbonée en C₁-C₈ linéaire ou ramifiée, pouvant former un ou plusieurs cycles carbonés comportant de 3 à 6 chaînons, et pouvant être saturée ou insaturée, dont un ou plusieurs atomes de carbone de la chaîne carbonée peuvent être remplacés par un atome d'oxygène, d'azote ou de soufre ou par un groupement SO₂, et dont les atomes de carbone peuvent être, indépendamment les uns des autres, substitués par un ou plusieurs atomes d'halogène ; R₅, R₆, R₇ et R₈ ne comportant pas de liaison peroxyde, ni de radicaux diazo ou nitroso.
- R₃, R₄, R₁₀, R₁₁, R₁₃, R₁₄ et R₁₅ représentent, identiques ou différents, un atome d'hydrogène, une chaîne hydrocarbonée en C₁-C₁₆, de préférence en C₁-C₈, linéaire ou ramifiée, pouvant former un ou plusieurs cycles carbonés comportant de 3 à 6 chaînons, et pouvant être saturée ou insaturée, dont un ou plusieurs atomes de carbone de la chaîne carbonée peuvent être remplacés par un atome d'oxygène, d'azote ou de soufre ou par un groupement SO₂, et dont les atomes de carbone peuvent être, indépendamment les uns des autres, substitués par un ou plusieurs atomes d'halogène ; R₃, R₄, R₁₀, R₁₁, R₁₃, R₁₄ et R₁₅ ne comportant pas de liaison peroxyde, ni de radicaux diazo ou nitroso.
- R₁₄ avec R₁₅ peuvent former un cycle aromatique carboné, tel qu'un phényle.

Les colorants de formule (I) peuvent être obtenus à partir de procédés de préparation connus et décrits par exemple dans les documents DE-19 721619, ou US-5 852 179.

Les exemples qui suivent servent à illustrer l'invention sans toutefois présenter un caractère limitatif.

### Exemple 1

On a préparé le colorant conforme à l'invention de formule suivante: 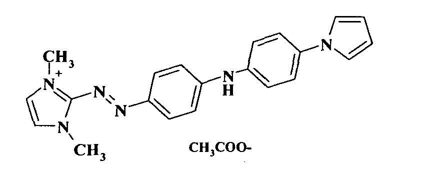 Acétate de 2-(4-amino-N-(4-(N-pyrrolophényl))-phénylazo)-1,3-diméthyl-3H-imidazol-1-ium.

Dans un ballon tout équipé, on a chargé 1 g (2.9 mmoles) de chlorure de 2-(4-amino-N-(4-aminophényl)-phénylazo)-1,3-diméthyl-3H-imidazol-1-ium et 100ml d'éthanol. On a ajouté au milieu réactionnel 1 ml de diméthoxytétrahydrofuranne (7.7 mmoles) et 4 ml d'acide acétique. Le mélange a été porté à 80°C pendant 35 minutes. Sous agitation, on a laissé revenir à température ambiante. Le milieu réactionnel a été versé dans 100 ml d'éther diisopropylique. Après filtration sur fritté et séchage sous vide à 30°C, on a récupéré 0.75 g d'acétate de 2-(4-amino-N-(4-(N-pyrrolophényl))-phénylazo)-1,3-diméthyl-3H-imidazol-1-ium sous forme de poudre violette avec un rendement de 62 %.
Les caractéristiques d'absorption du rayonnement visible par ce colorant sont les suivantes :
λₘₐₓ= 527 nm (EtOH)
ε = 50 000

### Analyses :

Masse ESI+ : m/z = 357 [M⁺]
RMN ¹H : (400MHz-CD₃OD) :
2,01 ppm, 3H, CH₃
4,07 ppm, 6H, 2 x CH₃
6,29 ppm, 2H pyrrole
7,17 ppm, 4H aromatiques
7,39 ppm, 2H aromatique
7,53 ppm, 2H aromatique
7,60 ppm, 2H imidazole
8,02 ppm 2H pyrrole

### Exemple 2

On a préparé le colorant conforme à l'invention de formule suivante: 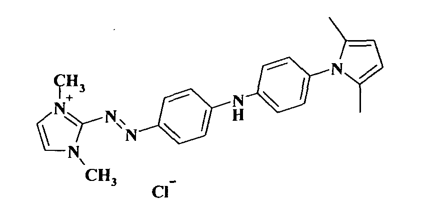 Chlorure de 2-(4-amino-N-(4-(N-(2,5-diméthy)-pyrrolophényl))-phénylazo)-1,3-diméthyl-3H-imidazol-1-ium.

Dans un ballon muni d'une colonne de distillation, on a dissous le chlorure de 2-(4-amino-N-(4-aminophényl)-phénylazo)-1,3-diméthyl-3H-imidazol-1-ium (0.75 g, 2,2 mmole) dans de l'éthanol anhydre (100 ml) ; on a ajouté de la 2,5-hexanedione (0.32 ml, 1.2 éq) et de l'acide paratoluènesulfonique en quantité catalytique (4 mg, 1 % catalytique). On a porté le milieu réactionnel à 130°C pendant 5 heures en ajoutant l'éthanol anhydre au fur et à mesure de la distillation pour ne pas assécher le milieu réactionnel.
Une fois la réaction terminée, on a laissé revenir à température ambiante.
Le milieu réactionnel a été versé dans 100 ml d'éther diisopropylique. Après filtration sur fritté et séchage sous vide à 30°C, on a récupéré 0.91 g de chlorure de 2-(4-amino-N-(4-(N-(2,5-diméthyl)-pyrrolophényl))-phénylazo)-1,3-diméthyl-3H-imidazol-1-ium sous forme de poudre violette avec un rendement de 98 %.
Les caractéristiques d'absorption du rayonnement visible par ce produit sont les suivantes :
λₘₐₓ = 527 nm (EtOH)
ε = 52 300

### Analyses :

Masse ESI+ : m/z = 385 [M⁺]
RMN ¹H : (400MHz-CD₃OD) :
2,02 ppm, 6H, 2 x CH₃
4,07 ppm, 6H, 2 x CH₃
5,80 ppm, 2H pyrrole
7,23 ppm, 4H aromatiques
7,42 ppm, 2H aromatique
7,59 ppm, 2H imidazole
8,02 ppm 2H aromatique

### Exemple 3

On a préparé le colorant conforme à l'invention de formule suivante: 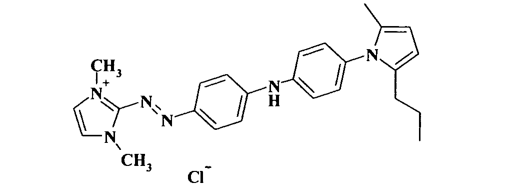 Chlorure de 2-(4-amino-N-(4-(N-(2-méthyl-5-propyl)-pyrrolophényl))-phénylazo)-1,3-diméthyl-3H-imidazol-1-ium.

Dans un ballon muni d'une colonne de distillation, on a dissous le chlorure de 2-(4-amino-N-(4-aminophényl)-phénylazo)-1,3-diméthyl-3H-imidazol-1-ium (0.75 g, 2,2 mmole) dans de l'éthanol anhydre (100 ml) ; on a ajouté de la 2,5-octanedione (0.4 g, 1.2 éq) et de l'acide paratoluènesulfonique en quantité catalytique (4 mg, 1 % catalytique). On a porté le milieu réactionnel à 130°C pendant 5 heures en ajoutant de l'éthanol anhydre au fur et à mesure de la distillation pour ne pas assécher le milieu réactionnel.
Une fois la réaction terminée, on a laissé revenir à température ambiante.
Le milieu réactionnel a été versé dans 100 ml d'éther diisopropylique. Après filtration sur fritté et séchage sous vide à 30°C, on a récupéré 0,5 g de chlorure de 2-(4-amino-N-(4-(N-(2-méthyl-5-propyl)-pyrrolophényl))-phénylazo)-1,3-diméthyl-3H- imidazol-1-ium sous forme de poudre violette avec un rendement de 51%.
Les caractéristiques d'absorption du rayonnement visible par ce produit sont les suivantes :
λₘₐₓ = 528 nm (EtOH)
ε = 53 900

### Analyses :

Masse ESI+ : m/z = 413 [M⁺]
RMN ¹H : (400MHz-CD₃OD) :
0.82 ppm, 3H, CH₃
1.43 ppm, 2H, CH₂
1.99 ppm, 3H, CH₃
2.33 ppm, 2H, CH₂
4,07 ppm, 6H, 2 x CH₃
5,81 ppm, 2H pyrrole
7,23 ppm, 4H aromatiques
7,42 ppm, 2H aromatique
7,59 ppm, 2H imidazole
8,02 ppm 2H aromatique

### Exemple 4 :

On a préparé le colorant conforme à l'invention de formule suivante: 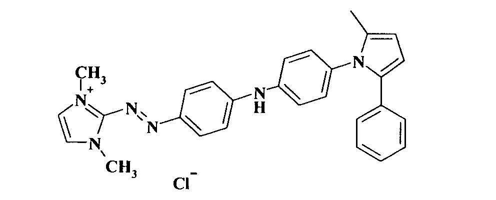 Chlorure de 2-(4-amino-N-(4-(N-(2-méthyl-5-phényl)-pyrrolophenyl))-phénylazo)-1,3-diméthyl-3H-imidazol-1-ium.

Dans un ballon muni d'une colonne de distillation, on a dissous le chlorure de 2-(4-amino-N-(4-aminophényl)-phénylazo)-1,3-diméthyl-3H-imidazol-1-ium (0.75 g, 2,2 mmole) dans de l'éthanol anhydre (100 ml) ; on a ajouté de la 1-phényl-1,4-pentanedione (0.46 g, 1.2 éq) et de l'acide paratoluènesulfonique en quantité catalytique (4 mg, 1 % catalytique). On a porté le milieu réactionnel à 130°C pendant 5 heures en ajoutant de l'éthanol anhydre au fur et à mesure de la distillation pour ne pas assécher le milieu réactionnel.
Une fois la réaction terminée, on a laissé revenir à température ambiante.
Le milieu réactionnel a été versé dans 100 ml d'éther diisopropylique. Après filtration sur fritté et séchage sous vide à 30°C, on a récupéré 0.8 g de chlorure de 2-(4-amino-N-(4-(N-(2-méthyl-5-phényl)-pyrrolophényl))-phénylazo)-1,3-diméthyl-3H-imidazol-1-ium sous forme de poudre violette avec un rendement de 76 %.
Les caractéristiques d'absorption du rayonnement visible par ce produit sont les suivantes :
λₘₐₓ= 530 nm (EtOH)
ε=55190

### Analyses :

Masse ESI+ : m/z = 483 [M⁺]
RMN ¹H : (400MHz-CD₃OD) :
2.13 ppm, 3H, CH₃
4,05 ppm, 6H, 2 x CH₃
6,01 ppm, 1 H pyrrole
6,25 ppm, 1 H pyrrole
7,30-7,04 ppm, 9H aromatiques
7,31 ppm, 2H aromatique
7,58 ppm, 2H imidazole
7,98 ppm 2H aromatique

### Exemple 5

On a préparé le colorant conforme à l'invention de formulé suivante: 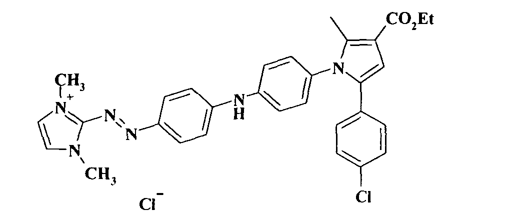 Chlorure de 2-(4-amino-N-(4-(N-[2-méthyl-3-carboxyéthyl-5-(4-chlorophényl)-pyrrolophényl)]-phénylazo)-1,3-diméthyl-3H-imidazol-1 -ium.

Dans un ballon muni d'une colonne de distillation, on a dissous le chlorure de 2-(4-amino-N-(4-aminophényle)-phénylazo)-1,3-diméthyl-3H-imidazol-1-ium (0.75 g, 2,2 mmole) dans de l'éthanol anhydre (100 ml) ; on a ajouté de la 1-phényl-1,4-pentanedione (0.46 g, 1.2 éq) et de l'acide paratoluènesulfonique en quantité catalytique (4 mg, 1 % catalytique). On a porté le milieu réactionnel à 130°C pendant 5 heures en ajoutant de l'éthanol anhydre au fur et à mesure de la distillation pour ne pas assécher le milieu réactionnel.
Une fois la réaction terminée, on a laissé revenir à température ambiante.
Le milieu réactionnel a été versé dans 100 ml d'éther diisopropylique. Après filtration sur fritté et séchage sous vide à 30°C, on a récupéré 1.3 g de chlorure de 2-(4-amino-N-(4-(N-[2-méthyl-3-carboxyéthyl-5-(4-chlorophényl)-pyrrolophényl)]-phénylazo)-1,3-diméthyl-3H-imidazol-1-ium sous forme de poudre violette avec un rendement de 99%.
Les caractéristiques d'absorption du rayonnement visible par ce produit sont les suivantes :
λₘₐₓ= 525 nm (EtOH)
ε = 52 000

### Analyses :

Masse ESI+ : m/z = 554 [M⁺]
RMN ¹H : (400MHz-CD₃OD) :
1.36 ppm, 3H, CH₃ (fonction ester)
2.40 ppm, 3H, CH₃
4,07 ppm, 6H, 2 x CH₃
4.28 ppm, 2H, CH₂ (fonction ester)
6,74 ppm, 1H pyrrole
7,07 ppm, 2H aromatique
7,23-7,16 ppm, 6H aromatiques
7,37 ppm, 2H aromatique
7,60 ppm, 2H imidazole
8,02 ppm 2H aromatique

### Exemples de teinture 6 à 10

On a préparé juste avant emploi les 5 compositions de teinture éclaircissantes suivantes : [quantités exprimées en grammes de matière active (MA) ]

| **EXEMPLE** | **6** | **7** | **8** | **9** | **10** |
|---|---|---|---|---|---|
| Colorant de formule (I) selon l'invention des exemples 1 à 5 | **Colorant exemple 1** **0,3** | **Colorant exemple 2** **0,2** | **Colorant exemple 3** **0,2** | **Colorant exemple 4** **0,2** | **Colorant exemple 5** **0,3** |
| Hydroxyéthylcellulose | 0,768 | 0,768 | 0,768 | 0,768 | 0,768 |
| Tensio actif non ionique : Alkyl(C8/C10 50/50) polyglucoside en solution aqueuse à 60%........................ | 6 | 6 | 6 | 6 | 6 |
| Alcool benzylique | 8 | 8 | 8 | 8 | 8 |
| Polyéthylène glycol (8OE)..... | 12 | 12 | 12 | 12 | 12 |
| Solution aqueuse d'ammoniaque q.s. pH........................ | 9 | 9 | 9 | 9 | 9 |
| Conservateurs | q.s | q.s | q.s | q.s | q.s |
| Eau déminéralisée...q.s.p. | 100 | 100 | 100 | 100 | 100 |

immédiatement avant emploi, on a mélangé une partie en poids de chacune de ces compositions colorantes avec une partie en poids de peroxyde d'hydrogène à 40 volumes. Puis on appliqué chacune de ces compositions sur des mèches de cheveux naturels à 90% de blancs.

On a laissé poser 30 minutes à température ambiante (environ 20°C), puis on a rincé les mèches à l'eau, on les a lavées au shampooing puis séchées.

La couleur obtenue a été évaluée dans le système de coloration MUNSELL.
Les nuances obtenues ont été réunies dans le tableau ci-dessous.

| | Valeurs MUNSELL Hue Value/Chroma | Reflet |
|---|---|---|
| EXEMPLE 6 | 3.9 RP 2,1 / 2.0 | violet |
| EXEMPLE 7 | 4.7 RP 2.3/3.0 | violet |
| EXEMPLE 8 | 6.4 RP 2.3 / 3.5 | violet |
| EXEMPLE 9 | 6.5 RP 2.5 / 3.8 | violet |
| EXEMPLE 10 | 9.4 RP 2.7/5.1 | violet |

## Revendications

1. Composition pour la teinture des fibres kératiniques humaines, et plus particulièrement des cheveux, comprenant dans un milieu cosmétiquement acceptable, au moins un colorant monoazoïque monocationique de formule (I) suivante :
**W**_{**1**}**―N=N―W**_{**2**}**―NW**_{**3**}**―W**_{**4**}**―W**_{**5**} **(I)**
dans laquelle
- W₁ représente un hétérocycle aromatique cationique à 5 ou 6 chaînons de formules (II) et (III) suivantes : 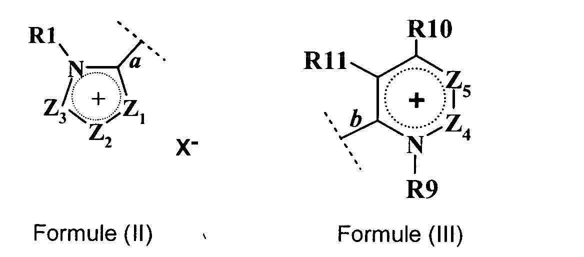
dans lesquelles :
- Z₁ représente un atome d'oxygène, un atome de soufre, un radical NR₂, ou un radical CR₃,
- Z₂ représente un atome d'azote ou un radical CR₄,
- Z₃ représente un radical NR₁₂ ou un radical CR₁₃,
- Z₄ représente un atome d'azote ou un radical CR₁₄,
- Z₅ représente un atome d'azote ou un radical CR₁₅,
Etant entendu que les formules (II) et (III) ne contiennent pas plus de deux hétéroatomes contigus ;
- la liaison a relie le cycle cationique à 5 chaînons de formule (II) à la fonction azoïque de la formule (I).
- la liaison b du cycle cationique à 6 chaînons de la formule (III) est reliée à la fonction azoïque de la formule (I).
- X- est un anion organique ou minéral.
- W₂ et W₄ (formule I) représentent, identiques ou différents, un groupe divalent aromatique carboné ou pyridinique de formules (IV) ou (V) suivantes : 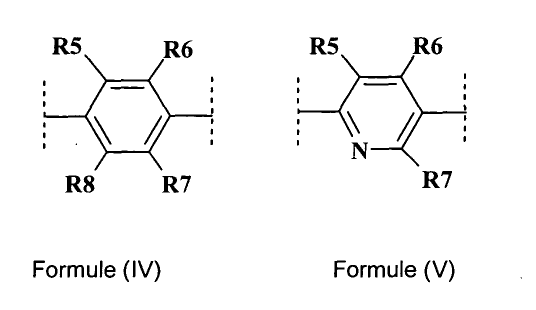
- W₃ représente un atome d'hydrogène ou un radical alkyle en C₁-C₆ éventuellement substitué par un ou plusieurs radicaux hydroxy, un ou plusieurs radicaux alcoxy, un ou plusieurs radicaux amino, un ou plusieurs radicaux monoalkylamino en C₁-C₄, un ou plusieurs radicaux dialkylamino en C₁-C₄,
- W₅ représente un radical hétéroaromatique azoté à 5 chaînons relié à W₄ par l'atome d'azote du cycle dudit radical hétéroaromatique, ce radical héréroaromatique étant choisi parmi les radicaux pyrazolyle, pyrrolyle, imidazolyle, triazolyle, thiadiazolyle, chacun de ces radicaux hétéroaromatiques pouvant être substitué par un ou plusieurs atomes d'hydrogène, de chlore ou de fluor, ou par un ou plusieurs radicaux alkyle en C₁-C₆, éventuellement substitués par un ou plusieurs radicaux hydroxy, alcoxy en C₁-C₄, (poly)-hydroxyalcoxy en C₂-C₄, amino, (di)alkylamino en C₁-C₄, (poly)hydroxyalkylamino en C₂-C₄, carboxyle, sulfonyle, alcoxycarbonyle en C₁-C₄, thioether en C₁-C₄ ; ou par un ou plusieurs radicaux phényle éventuellement substitués par un ou plusieurs radicaux choisis parmi les radicaux hydroxy, alcoxy en C₁-C₂, amino, (di)-alkylamino en C₁-C₂, carboxy, sulfonyle, alkyle en C₁-C₄, halogène, thioéther en C₁-C₂,
- R₁, R₂, R₉, R₁₂ représentent, identiques ou différents, un radical phényle éventuellement substitué ou un radical alkyle en C₁-C₈, éventuellement substitué par un ou plusieurs radicaux choisis dans le groupe formé par les radicaux hydroxy, alcoxy en C₁-C₂, (poly)-hydroxyalcoxy en C₂-C₄, amino, (di)alkylamino en C₁-C₂,
- R₅, R₆, R₇ et R₈ représentent, identiques ou différents, un atome d'hydrogène ; un atome de chlore ; un atome de brome ; une chaîne hydrocarbonée en C₁-C₈ linéaire ou ramifiée, pouvant former un ou plusieurs cycles carbonés comportant de 3 à 6 chaînons, et pouvant être saturée ou insaturée, dont un ou plusieurs atomes de carbone de la chaîne carbonée peuvent être remplacés par un atome d'oxygène, d'azote ou de soufre ou par un groupement SO₂, et dont les atomes de carbone peuvent être, indépendamment les uns des autres, substitués par un ou plusieurs atomes d'halogène ; R₅, R₆, R₇ et R₈ ne comportant pas de liaison peroxyde, ni de radicaux diazo ou nitroso.
- R₃, R₄, R₁₀, R₁₁, R₁₃, R₁₄ et R₁₅ représentent, identiques ou différents, un atome d'hydrogène, une chaîne hydrocarbonée en C₁-C₁₆, de préférence en C₁-C₈, linéaire ou ramifiée, pouvant former un ou plusieurs cycles carbonés comportant de 3 à 6 chaînons, et pouvant être saturée ou insaturée, dont un ou plusieurs atomes de carbone de la chaîne carbonée peuvent être remplacés par un atome d'oxygène, d'azote ou de soufre ou par un groupement SO₂, et dont les atomes de carbone peuvent être, indépendamment les uns des autres, substitués par un ou plusieurs atomes d'halogène ; R₃, R₄, R₁₀, R₁₁, R₁₃, R₁₄ et R₁₅ ne comportant pas de liaison peroxyde, ni de radicaux diazo ou nitroso.
- R₄ avec R₁₃ et R₁₄ avec R₁₅ peuvent former un cycle aromatique carboné, tel qu'un phényle.

2. Composition selon la revendication 1 dans laquelle W₃ (formule I) représente un atome d'hydrogène ou un radical alkyle en C₁-C₄ éventuellement substitué par un ou plusieurs radicaux choisis parmi les radicaux hydroxy, alcoxy en C₁-C₂, amino, (di)alkylamino en C₁-C₂ .

3. Composition selon la revendication 2 dans laquelle W₃ (formule I) représente un atome d'hydrogène ou un radical méthyle, éthyle, 2-hydroxyéthyle.

4. Composition selon la revendication 3 dans laquelle W₃ (formule I) représente un atome d'hydrogène.

5. Composition selon l'une quelconque des revendications précédentes dans laquelle W₅ (formule I) est choisi parmi les cycles pyrazolyle, pyrrolyle, imidazolyle, et plus particulièrement encore parmi les cycles pyrrolyle, imidazolyle.

6. Composition selon la revendication 5 dans laquelle W₅ (formule I) est un cycle hétéroaromatique azoté à 5 chaînons substitué par un ou plusieurs radicaux alkyle en C₁-C₆ éventuellement substitués par un hydroxy, alcoxy en C₁-C₄, amino, mono ou dialkylamino en C₁-C₄ ; un atome de chlore ou de fluor ; un radical alcoxycarbonyle en C₁-C₄ ; un radical phényle éventuellement substitué par un hydroxy, alcoxy en C₁-C₂, amino, (di)alkylamino en C₁-C₂, un atome de brome ou de chlore ; un radical dialkylamino en C₁-C₂, un radical alcoxy en C₁-C₂.

7. Composition selon la revendication 6 dans laquelle W₅ (formule I) est choisi parmi les radicaux pyrrolyle, imidazolyle éventuellement substitués par un à deux radicaux méthyle, éthyle, propyle, phényle, 4-chlorophényle, éthoxycarbonyle.

8. Composition selon l'une quelconque des revendications précédentes dans laquelle les substituants R₁, R₂, R₉, R₁₂ représentent, identiques ou différents, un radical alkyle en C₁-C₄ éventuellement substitué par un ou plusieurs radicaux choisis parmi les radicaux hydroxy, alcoxy en C₁-C₂, amino, (di)alkylamino en C₁-C₂.

9. Composition selon la revendication 8 dans laquelle R₁, R₂, R₉, R₁₂ représentent, identiques ou différents, un radical méthyle, éthyle, propyle, 2-hydroxyéthyle.

10. Composition selon l'une quelconque des revendications précédentes dans laquelle les substituants R₅, R₆, R₇ et R₈ représentent, identiques ou différents, un atome d'hydrogène, un radical méthyle, éthyle, isopropyle, méthoxyméthyle, hydroxyméthyle, 1-carboxyméthyle, 1-aminométhyle, 2-carboxyéthyle, 2-hydroxyéthyle, 3-hydroxypropyle, 1,2-dihydroxyéthyle, 1-hydroxy-2-aminoéthyle, 2-hydroxy-1-aminoéthyle, méthoxy, éthoxy, 2-hydroxyéthyloxy, 2-aminoéthyloxy.

11. Composition selon la revendication 10 dans laquelle R₅, R₆, R₇ et R₈, identiques ou différents, représentent un atome d'hydrogène, un radical méthyle, hydroxyméthyle, 2-hydroxyéthyle, 1,2-dihydroxyéthyle, méthoxy, 2-hydroxyéthyloxy.

12. Composition selon la revendication 11 dans laquelle R₅, R₆, R₇ et R₈, identiques ou différents, représentent un atome d'hydrogène, un radical méthyle, un radical méthoxy.

13. Composition selon l'une quelconque des revendications précédentes dans laquelle les substituants R₃, R₄, R₁₀, R₁₁, R₁₃, R₁₄ et R₁₅, identiques ou différents, représentent un atome d'hydrogène ; un radical alkyle en C₁-C₄ linéaire ou ramifié, éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁-C₂ ; un radical phényle éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁-C₂, ou un atome d'halogène tel que chlore, fluor ou brome ; un radical sulfonylamino ; un radical alcoxy en C₁-C₂ ; un radical (poly)-hydroxyalcoxy en C₂-C₄; un radical amino ; un radical (di)alkylamino en C₁-C₂ ; un radical (poly)-hydroxyalkylamino en C₂-C₄.

14. Composition selon la revendication 13 dans laquelle, R₃, R₄, R₁₀, R₁₁, R₁₃, R₁₄ et R₁₅ représentent, identiques ou différents, un atome d'hydrogène, un radical alkyle en C₁-C₄ éventuellement substitué par un ou plusieurs radicaux choisis parmi les radicaux hydroxy, amino, (di)alkylamino en C₁-C₂ ; un radical alcoxy en C₁-C₂ ; un radical amino ; un radical (di)alkylamino en C₁-C₂ ; un radical (poly)-hydroxyalkylamino en C₂-C₄.

15. Composition selon la revendication 14 dans laquelle R₃, R₄, R₁₀, R₁₁, R₁₃, R₁₄ et R₁₅ représentent, identiques ou différents, un atome d'hydrogène, un radical méthyle, phényle, 2-hydroxyméthyle, méthoxy, éthoxy, 2-hydroxyéthyloxy, amino, méthylamino, diméthylamino, 2-hydroxyéthylamino.

16. Composition selon l'une quelconque des revendications précédentes dans laquelle Z1 [formule (II)] désigne NR₂.

17. Composition selon l'une quelconque des revendications précédentes dans laquelle Z2 [formule (II)] désigne CR₄.

18. Composition selon l'une quelconque des revendications précédentes dans laquelle Z3 [formule (II)] désigne CR₁₃.

19. Composition selon l'une quelconque des revendications précédentes dans laquelle Z4 [formule (III)] désigne CR₁₄.

20. Composition selon l'une quelconque des revendications précédentes dans laquelle Z5 [formule (III)] désigne CR₁₅.

21. Composition selon l'une quelconque des revendications précédentes **caractérisée par le fait que** le colorant monoazoïque monocationique de formule (I) est choisi dans le groupe formé par les :
2-(4-amino-N-(4-(N-pyrrolophényl))-phénylazo)-1,3-diméthyl-3H-imidazol-1-ium,
2-(4-amino-N-(4-(N-(2,5-diméthyl)-pyrrolophényl))-phénylazo)-1,3-diméthyl-3H-imidazol-1-ium,
2-(4-amino-N-(4-(N-(2-méthyl-5-propyl)-pyrrolophényl))-phénylazo)-1,3-diméthyl-3H-imidazol-1-ium,
2-(4-amino-N-(4-(N-(2-méthyl-5-phényl)-pyrrolophényl))-phénylazo)-1,3-diméthyl-3H-imidazol-1-ium,
2-(4-amino-N-(4-(N-[2-méthyl-3-carboxyéthyl-5-(4-chlorophényl)-pyrrolophényl)]-phénylazo)-1,3-diméthyl-3H-imidazol-1 -ium,
chacun de ces colorants étant associé à un anion X⁻.

22. Composition selon l'une quelconque des revendications précédentes dans laquelle X- désigne un halogénure, un hydroxyde, un sulfate, un hydrogénosulfate, un alkyl(C₁-C₆)sulfate, un acétate, un tartrate, un oxalate, un alkyl(C₁-C₆)sulfonate, un arylsulfonate substitué ou non substitué par un radical alkyle en C₁-C₄.

23. Composition selon l'une quelconque des revendications précédentes, **caractérisée par le fait que** le colorant monoazoïque monocationique de formule (I) est présent à une concentration variant de 0,001 à 5% en poids par rapport au poids total de la composition tinctoriale, et de préférence de 0,05 à 2%.

24. Composition selon l'une quelconque des revendications 1 à 23 comprenant en outre au moins un colorant direct différent de ceux de formule (I), choisi parmi les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs méthiniques, les colorants directs triarylméthaniques, les colorants directs indoaminiques et les colorants directs naturels.

25. Composition selon l'une quelconque des revendications précédentes comprenant en outre un agent oxydant, de préférence le peroxyde d'hydrogène.

26. Composition selon l'une quelconque des revendications 1 à 25 comprenant de plus une base d'oxydation.

27. Composition selon la revendication 26 dans laquelle la base d'oxydation est choisie parmi les paraphénylènediamines, les bis-phénylalkylènediamines, les para-aminophénols, les ortho-aminophénols, les bases hétérocycliques, et leurs sels d'addition avec un acide.

28. Composition selon l'une quelconque des revendications 26 à 27 comprenant en outre au moins un coupleur.

29. Composition selon la revendication 28 dans laquelle le coupleur est choisi parmi les métaphénylènediamines, les méta-aminophénols, les métadiphénols, les coupleurs naphtaléniques et les coupleurs hétérocycliques et leur sel d'addition avec un acide.

30. Procédé de teinture directe des fibres kératiniques humaines et plus particulièrement des cheveux, **caractérisé en ce qu'**on applique sur les fibres au moins une composition tinctoriale telle que définie à l'une quelconque des revendications 1 à 24.

31. Procédé selon la revendication 30 dans lequel la composition tinctoriale contient un agent oxydant.

32. Procédé selon la revendication 31 dans lequel l'agent oxydant est mélangé au moment de l'emploi à la composition tinctoriale.

33. Procédé selon la revendication 32 dans lequel l'agent oxydant est appliqué sur les fibres sous forme de composition oxydante simultanément ou séquentiellement à la composition tinctoriale.

34. Procédé de teinture d'oxydation des fibres kératiniques humaines et plus particulièrement des cheveux, **caractérisé en ce qu'**on applique sur les fibres au moins une composition tinctoriale telle que définie à l'une quelconque des revendications 1 à 24, comprenant de plus au moins une base d'oxydation et optionnellement au moins un coupleur, en présence d'un agent oxydant.

35. Procédé selon la revendication 34 dans lequel l'agent oxydant est mélangé au moment de l'emploi à la composition tinctoriale.

36. Procédé selon la revendication 34 dans lequel l'agent oxydant est appliqué sur les fibres sous forme de composition oxydante simultanément ou séquentiellement à la composition tinctoriale.

37. Dispositif à plusieurs compartiments ou "kit" de teinture à plusieurs compartiments pour la teinture des fibres kératiniques humaines, dans lequel un premier compartiment contient une composition telle que définie à l'une quelconque des revendications 1 à 24 et 26 à 29 et un deuxième compartiment contient une composition oxydante.

38. Composés monoazoïques monocationiques de formule (I) telle que définie à l'une quelconque des revendications 1 à 24 et pour laquelle, quand W₅ désigne imidazole, et que W₁ désigne benzimidazole, W₃ est différent de l'hydrogène si W₄ désigne phényle.

39. Composés monoazoïques monocationiques de formule (I)
**W**_{**1**}**― N=N―W**_{**2**}**― NW**_{**3**} **―W4― W**_{**5**} **(I)**
dans laquelle :
- W₁ représente un hétérocycle aromatique cationique à 5 ou 6 chaînons de formules (II) et (III) suivantes : 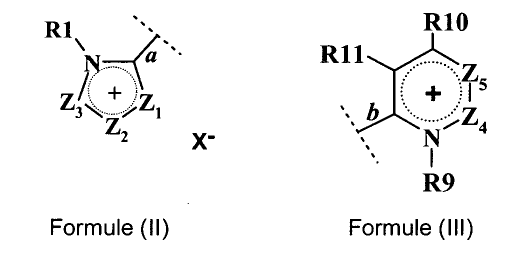
dans lesquelles :
- Z₁ représente un atome d'oxygène, un atome de soufre, un radical NR₂, ou un radical CR₃,
- Z₂ représente un atome d'azote ou un radical CR₄,
- Z₃ représente un radical NR₁₂ ou un radical CR₁₃,
- Z₄ représente un atome d'azote ou un radical CR₁₄,
- Z₅ représente un atome d'azote ou un radical CR₁₅,
Etant entendu que les formules (II) et (III) ne contiennent pas plus de deux hétéroatomes contigus ;
- La liaison a relie le cycle cationique à 5 chaînons de formule (II) à la fonction azoïque de la formule (I).
- la liaison b du cycle cationique à 6 chaînons de la formule (III) est reliée à la fonction azoïque de la formule (I).
- X- est un anion organique ou minéral.
- W₂ et W₄ (formule I) représentent, identiques ou différents, un groupe divalent aromatique carboné ou pyridinique de formules (IV) ou (V) suivantes : 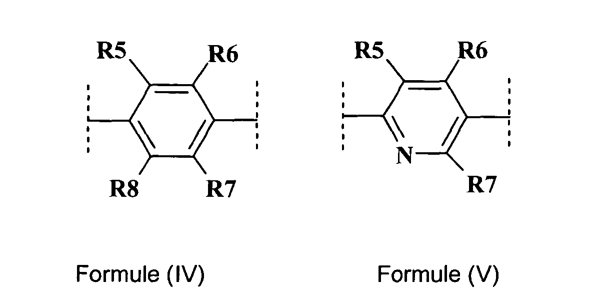
- W₃ représente un atome d'hydrogène ou un radical alkyle en C₁-C₆ pouvant éventuellement être substitué par un ou plusieurs radicaux hydroxy, un ou plusieurs radicaux alcoxy, un ou plusieurs radicaux amino, un ou plusieurs radicaux monoalkylamino en C₁-C₄, un ou plusieurs radicaux dialkylamino en C₁-C₄,
- W₅ représente un radical hétéroaromatique azoté à 5 chaînons relié à W₄ par l'atome d'azote du cycle dudit radical hétéroaromatique, ce radical héréroaromatique étant choisi parmi les radicaux pyrazolyle, pyrrolyle, imidazolyle, triazolyle, thiadiazolyle, chacun de ces radicaux hétéroaromatiques pouvant être substitué par un ou plusieurs atomes d'hydrogène, de chlore ou de fluor, ou par un ou plusieurs radicaux alkyle en C₁-C₆, éventuellement substitués par un ou plusieurs radicaux hydroxy, alcoxy en C₁-C₄, (poly)-hydroxyalcoxy en C₂-C₄, amino, (di)alkylamino en C₁-C₄, (poly)hydroxyalkylamino en C₂-C₄, carboxyle, sulfonyle, alcoxycarbonyle en C₁-C₄, thioether en C₁-C₄ ; ou par un ou plusieurs radicaux phényle éventuellement substitués par un ou plusieurs radicaux choisis parmi les radicaux hydroxy, alcoxy en C₁-C₂, amino, (di)-alkylamino en C₁-C₂, carboxy, sulfonyle, alkyle en C₁-C₄, halogène, thioéther en C₁-C₂,
- R₁, R₂, R₉, R₁₂ représentent, identiques ou différents, un radical phényle éventuellement substitué ou un radical alkyle en C₁-C₈, éventuellement substitué par un ou plusieurs radicaux choisis dans le groupe formé par les radicaux hydroxy, alcoxy en C₁-C₂, (poly)-hydroxyalcoxy en C₂-C₄, amino, (di)alkylamino en C₁-C₂ ,
- R₅, R₆, R₇ et R₈ représentent, identiques ou différents, un atome d'hydrogène ; un atome de chlore ; un atome de brome ; une chaîne hydrocarbonée en C₁-C₈ linéaire ou ramifiée, pouvant former un ou plusieurs cycles carbonés comportant de 3 à 6 chaînons, et pouvant être saturée ou insaturée, dont un ou plusieurs atomes de carbone de la chaîne carbonée peuvent être remplacés par un atome d'oxygène, d'azote ou de soufre ou par un groupement SO₂, et dont les atomes de carbone peuvent être, indépendamment les uns des autres, substitués par un ou plusieurs atomes d'halogène ; R₅, R₆, R₇ et R₈ ne comportant pas de liaison peroxyde, ni de radicaux diazo ou nitroso.
- R₃, R₄, R₁₀, R₁₁, R₁₃, R₁₄ et R₁₅ représentent, identiques ou différents, un atome d'hydrogène, une chaîne hydrocarbonée en C₁-C₁₆, de préférence en C₁-C₈, linéaire ou ramifiée, pouvant former un ou plusieurs cycles carbonés comportant de 3 à 6 chaînons, et pouvant être saturée ou insaturée, dont un ou plusieurs atomes de carbone de la chaîne carbonée peuvent être remplacés par un atome d'oxygène, d'azote ou de soufre ou par un groupement SO₂, et dont les atomes de carbone peuvent être, indépendamment les uns des autres, substitués par un ou plusieurs atomes d'halogène ; R₃, R₄, R₁₀, R₁₁, R₁₃, R₁₄ et R₁₅ ne comportant pas de liaison peroxyde, ni de radicaux diazo ou nitroso.
- R₁₄ avec R₁₅ peuvent former un cycle aromatique carboné, tel qu'un phényle.

40. Utilisation des composés monoazoïques monocationiques de formule (I) telle que définie selon l'une quelconque des revendications 1 à 23, à titre de colorants directs, dans une, ou pour la préparation d'une composition de teinture des fibres kératiniques humaines et plus particulièrement des cheveux.
